# EUROPÄISCHE PATENTSCHRIFT

(11) **EP 1 966 116 B1**
(45) Veröffentlichungstag und Bekanntmachung des Hinweises auf die Patenterteilung: **06.06.2012**
(21) Anmeldenummer: 06830500.2
(22) Anmeldetag: 11.12.2006
(51) Int. Cl.: C07C 51/43, C07C 57/05

(54) **VERFAHREN DER HETEROGEN KATALYSIERTEN PARTIELLEN GASPHASENOXIDATION VON PROPYLEN ZU ACRYLSÄURE**
METHOD FOR THE HETEROGENEOUSLY CATALYSED PARTIAL GAS PHASE OXIDATION OF PROPYLENE TO FORM ACRYLIC ACID
PROCEDE D'OXYDATION EN PHASE GAZEUSE HETEROGENE ET PARTIELLEMENT CATALYSEE DE PROPYLENE EN ACIDE ACRYLIQUE

(30) Priorität: 22.12.2005 DE 102005062010; 22.12.2005 US 752362 P
(43) Veröffentlichungstag der Anmeldung: 10.09.2008
(73) Patentinhaber: BASF SE, 67056 Ludwigshafen (DE)
(72) Erfinder: DIETERLE, Martin, 67063 Ludwigshafen (DE); HEILEK, Jörg, 69245 Bammental (DE); MÜLLER-ENGEL, Klaus, Joachim, 76297 Stutensee (DE)
(86) Internationale Anmeldenummer: PCT/EP2006/069526
(87) Internationale Veröffentlichungsnummer: WO 2007/074044

(56) Entgegenhaltungen:
- WO-A-2006/002703
- DE-A1- 10 313 208
- DE-A1- 19 902 562

## Beschreibung

Vorliegende Erfindung betrifft ein Verfahren der heterogen katalysierten partiellen Gasphasenoxidation von Propylen zu Acrylsäure, bei dem man in einer ersten Reaktionszone ein Propylen, molekularen Sauerstoff und wenigstens ein inertes Verdünnungsgas enthaltendes Reaktionsgasausgangsgemisch 1, das den molekularen Sauerstoff und das Propylen in einem molaren Verhältnis O₂ : C₃H₆ ≥ 1 enthält, zunächst in einer ersten Reaktionsstufe bei erhöhter Temperatur so durch wenigstens ein erstes Katalysatorbett führt, dessen Katalysatoren als Aktivmasse wenigstens ein Mo, Fe und Bi enthaltendes Multimetalloxid aufweisen, dass der Propylenumsatz bei einmaligem Durchgang durch das Katalysatorbett ≥ 90 mol-% und die damit einhergehende Selektivität S^{AC} der Acroleinbildung sowie der Acrylsäurenebenproduktbildung zusammen ≥ 80 mol-% betragen, die Temperatur des die erste Reaktionsstufe verlassenden Produktgasgemischs 1 durch direkte Kühlung, oder durch indirekte Kühlung, oder durch direkte und indirekte Kühlung gegebenenfalls verringert und dem Produktgasgemisch 1 gegebenenfalls Sekundärgas in Form von molekularem Sauerstoff, oder Inertgas, oder molekularem Sauerstoff und Inertgas zugibt, und danach das Produktgasgemisch 1 als Acrolein, molekularen Sauerstoff und wenigstens ein inertes Verdünnungsgas enthaltendes Reaktionsgasausgangsgemisch 2, das den molekularen Sauerstoff und das Acrolein in einem molaren Verhältnis O₂ : C₃H₄O ≥ 0,5 enthält, in einer zweiten Reaktionsstufe bei erhöhter Temperatur so durch wenigstens ein zweites Katalysatorbett führt, dessen Katalysatoren als Aktivmasse wenigstens ein Mo und V enthaltendes Multimetalloxid aufweisen, dass der Acroleinumsatz bei einmaligem Durchgang durch das Katalysatorbett ≥ 90 mol-% und die Selektivität S^{AA} der über beide Reaktionsstufen bilanzierten Acrylsäurebildung, bezogen auf umgesetztes Propylen, ≥ 70 mol-% beträgt, anschließend aus dem in der zweiten Reaktionsstufe gebildeten Produktgasgemisch 2 die darin enthaltene Acrylsäure in einer ersten Trennzone in die kondensierte Phase überführt und danach in einer zweiten Trennzone die Acrylsäure aus der kondensierten Phase durch Anwendung wenigstens eines thermischen Trennverfahrens abtrennt.

Acrylsäure ist als ein Partialoxidationsprodukt des Propylen ein bedeutendes Monomeres, das als solches oder in Form seiner Alkylester zur Erzeugung von z. B. als Klebstoffe geeigneten oder Wasser superabsorbierenden Polymerisaten Verwendung findet (vgl. z. B. WO 02/055469 und WO 03/078378).

Die Herstellung von Acrylsäure nach einem eingangs dieser Schrift beschriebenen Verfahren ist bekannt (vgl. z. B. DE-A 102 45 585, WO 03/011804, DE-A 101 31 297, WO 01/96270), DE 103 13 208.

Das für diese Verfahrensweise als Ausgangssubstanz benötigte Propylen wird dem Reaktionsgasausgangsgemisch 1 üblicherweise als rohes Propylen (auch "Roh-Propylen") zugesetzt. Im Unterschied zu chemisch reinem Propylen enthält Roh-Propylen auch von Propylen chemisch verschiedene Bestandteile (Verunreinigungen), die auf das Roh-Propylen bezogen bis zu 10 Vol.-% und mehr betragen können. Beispielsweise kann Roh-Propylen auch das Produktgasgemisch einer heterogen katalysierten partiellen Propandehydrierung sein (vgl. DE-A 102 45 585 und DE-A 10 2005 022 798). Grundsätzlich ist es möglich, alle in Roh-Propylen enthaltenen Verunreinigungen vom darin enthaltenen Propylen abzutrennen (vgl. z. B. DE-A 35 21 458 und DE-A 102 45 585). Dies ist jedoch dann nicht erforderlich, wenn sich die Verunreinigungen im Rahmen der heterogen katalysierten Partialoxidation des Propylen zu Acrylsäure inert verhalten. Ist letztere Eigenschaft gegeben, wirken die Verunreinigungen im Reaktionsgasausgangsgemisch 1 in einfacher Weise als inerte Verdünnungsgase (vgl. WO 01/96270 und DE-A 33 13 573). Darunter werden in dieser Schrift ganz allgemein solche Gase verstanden, die im Verlauf der Partialoxidation, jeweils für sich, zu wenigstens 95 mol-%, bevorzugt zu wenigstens 97 mol-% und ganz besonders bevorzugt zu 99 mol-% oder mehr chemisch unverändert erhalten bleiben. Im Rahmen der Überführung der Acrylsäure aus dem Produktgasgemisch 2 in die kondensierte Phase verbleiben diese inerten Gase üblicherweise als Restgas in der Gasphase und können so nach der Partialoxidation in vergleichsweise einfacherer Weise vom Zielprodukt abgetrennt werden, als dies im Rahmen einer Abtrennung vom Propylen vorab der Partialoxidation der Fall wäre. Als solche Inertgase wurden bisher in der Fachliteratur im Bezug auf die Partialoxidation von Propylen zu Acrylsäure die Propane angesehen. Die Überlegungen gehen diesbezüglich sogar soweit, Propylen als Rohstoff zur Herstellung von Acrylsäure durch Propan als solchen Rohstoff zu ersetzen. In diesem Fall wird Propan in einem ersten Schritt partiell zu Propylen dehydriert und nachfolgend das im ersten Schritt gebildete Propylen im Beisein des nicht umgesetzten Propan heterogen katalysiert zu Acrylsäure partialoxidiert (vgl. WO 01/96270). Normalerweise bildet Propan in einem solchermaßen resultierenden Reaktionsgasausgangsgemisch 1 sogar den Hauptbestandteil. Durch Rückführung des bei der Kondensation des Zielproduktes aus dem Produktgasgemisch verbleibenden, das nicht umgesetzte Propan enthaltenden Restgases in die Dehydrierung und/oder Partialoxidation kann das Propan solchermaßen schließlich vollständig in Acrylsäure überführt werden (vgl. z. B. DE-A 102 45 585, DE-A 10 2005 009 885, DE-A 10 2005 010 111). Zwar kann eine verschwindend geringe Menge des Propan (größenordnungsgemäßig auf seine Einsatzmenge bezogen 0,01 Gew.-%) zu Propionsäure (sie ist bereits alleine aufgrund ihres auch in kleinsten Mengen unangenehmen Geruches sowie wegen ihres radikalischen Polymerisationsunvermögens ein unerwünschter Acrylsäurebegleiter) umgesetzt werden, doch lässt sich einer solchermaßen geringen Nebenproduktumsetzung z. B. dadurch begegnen, dass man das Propan enthaltende Reaktionsgasausgangsgemisch 1 zusätzlich mit einem von Propan verschiedenen inerten Verdünnungsgas (z. B. N₂, H₂O, CO₂, Edelgas, Gemische dieser Gase etc.) verdünnt (vgl. z. B. WO 01/96270). Die vorbeschriebenen Überlegungen sind jedoch dann nicht mehr zutreffend, wenn sich die Propylenverunreinigung in einer heterogen katalysierten partiellen Oxidation desselben zu Acrylsäure nicht inert verhält, sondern in nennenswerten Anteilen zu einem Nebenprodukt der Acrylsäurebildung umgesetzt wird. Dies ist darauf zurückzuführen, dass das gebildete Nebenprodukt normalerweise nicht als Zielproduktverunreinigung mit selbigem ausgelassen werden kann. Vielmehr wirken in vielen Fällen auch geringfügige Zielproduktverunreinigungen mit Blick auf die angestrebte Zielproduktverwendung als störend (z. B. bei einer Verwendung der Acrylsäure zur Herstellung von Polyacrylsäuren und/oder deren Teil- und/oder vollneutralisierten Alkalisalzen, die überwiegend als Wasser superabsorbierende Materialien im Hygienebereich zum Einsatz kommen; oder bei einer Verwendung der Acrylsäure zur Herstellung ihrer Alkylester und der Verwendung der letzteren zur Herstellung von als Klebstoffe geeigneten Polymerisaten) und müssen dann in einer zweiten Trennzone unter Anwendung wenigstens eines thermischen Trennverfahrens von der in der ersten Trennzone gebildeten, das Zielprodukt in kondensierter Form enthaltenden Phase vom Zielprodukt abgetrennt werden (bzw. umgekehrt). Eine solche Abtrennung kann vergleichsweise aufwendig sein. In solchen Fällen wird man dann zweckmäßigerweise versuchen, die entsprechende Propylenverunreinigung vorab der Partialoxidation abzutrennen.

In vielen Fällen wird auch parallel vorgegangen. D. h., eine Teilmenge der Propylenverunreinigung wird vorab der Propylenpartialoxidation abgetrennt, und die verbliebene Teilmenge wird nach durchgeführter Propylenpartialoxidation in der Trennzone 2 mittels wenigstens eines thermischen Trennverfahrens als Acrylsäurenebenprodukt von der Acrylsäure abgetrennt (bzw. umgekehrt).

Unter thermischen Trennverfahren sollen dabei solche Verfahren verstanden werden, bei denen wenigstens zwei voneinander verschiedene stoffliche Phasen (z. B. flüssig/flüssig; gasförmig/flüssig; fest/flüssig; gasförmig/fest etc.) erzeugt und miteinander in Kontakt gebracht werden. Aufgrund des zwischen den Phasen bestehenden Ungleichgewichts findet zwischen denselben ein Wärme- und Stoffaustausch statt, der letztlich die gewünschte Auftrennung (Abtrennung) bedingt. Die Bezeichnung thermische Trennverfahren reflektiert dabei, dass es entweder des Entzugs oder der Zufuhr von Wärme bedarf, um die Ausbildung der stofflichen Phasen zu erzeugen und/oder dass der Entzug oder die Zufuhr von thermischer Energie den Stoffaustausch begünstigt bzw. aufrechterhält.

Thermische Trennverfahren im Sinne der vorliegenden Erfindung sind daher Destillationen, Rektifikationen, Kristallisationen, Extraktionen, azeotrope Destilationen, azeotrope Rektifikationen, die Strippung, die Desorption etc. (vgl. auch WO 04/063138).

Kristallisative thermische Trennverfahren gelten als besonders investitionsintensiv und werden daher in der Regel zu vermeiden gesucht.

In überraschender Weise wurde nun im Rahmen eigener Arbeiten gefunden, dass Cyclopropan, ein nicht seltener Begleiter von Propylen in Roh-Propylen, im Rahmen einer wie eingangs beschriebenen heterogen katalysierten Partialoxidation von Propylen zu Acrylsäure kein Inertgas bildet. Beim Erhitzen auf 100 bis 200°C in Gegenwart von Katalysatoren (z. B. Pt) isomerisiert sich Cyclopropan zwar zu Propylen (z. B. Lehrbuch der Organischen Chemie, Beyer · Walter, Hirzel Verlag Stuttgart, Seite 390, 1991). Im Verlauf einer wie eingangs beschriebenen heterogen katalysierten Partialoxidation von Propylen zu Acrylsäure verhält es sich jedoch gänzlich anders als Propylen und reagiert nicht wie letzteres nahezu ausschließlich zu Acrylsäure, sondern in völlig unerwarteter Weise in hohem und in überraschender Weise erheblichem Umfang zu Propionsäure. Die Aufgabe der vorliegenden Erfindung bestand daher darin, mit diesem überraschenden Zufallsbefund im Kontext mit einer Herstellung von z. B. möglichst an Propionsäure armer Acrylsäure über den Weg einer zweistufigen heterogen katalysierten Partialoxidation von Propylen zu Acrylsäure in vorteilhafter Weise umzugehen. Dies auch vor dem Hintergrund, dass vorgenannte Isomerisierung zu Propylen ein geeigneter Weg sein könnte, das Cyclopropan vorab der Partialoxidation zu beseitigen. Prinzipiell können Propylen und Cyclopropan aber auch rektifikativ voneinander getrennt werden, sind ihre Siedepunkte bei Normaldruck (1 bar) doch ausreichend voneinander verschieden (Sdp. Propylen = -47°C; Sdp. Cyclopropan = -32,8°C). Vorgenannte Fragestellung und ihre Beantwortung ist vor allem dann von besonderem Interesse, wenn wenigstens eine Teilmenge des in der Trennzone 1 verbleibenden Restgases, das in der Partialoxidation nicht vollständig umgesetztes Cyclopropan enthalten würde, wenigstens teilweise als Kreisgas in die Partialoxidation als Bestandteilt des Reaktionsgasausgangsgemischs 1 rückgeführt wird, ginge mit einer solchen Kreisgasführung im kontinuierlichen Betrieb doch eine Aufpegelung des Cyclopropan im Rekationsgasausgangsgemisch 1 einher.

Als Lösung der erfindungsgemäßen Aufgabe wurde nun gefunden, dass es wenigstens bis zu Cyclopropangehalten von 3 mol-%, bezogen auf im Reaktionsgasausgangsgemisch 1 enthaltenes Propylen, zweckmäßig ist, das Cycloalkan als Verunreinigung im Roh-Propylen zu belassen und die aus selbigem in der Partialoxidation gebildete Propionsäure dadurch vom Zielprodukt Acrylsäure abzutrennen, dass das wenigstens eine thermische Trennverfahren in der zweiten Trennzone (in der Trennzone 2) wenigstens eine kristallisative Abtrennung von Acrylsäure umfasst (aufgrund vergleichbarer Kondensationspunkte (bei 1 bar Acrylsäure: 141°C, Propionsäure: 141,35°C) wird die Propionsäure in der Trennzone 1 normalerweise gemeinsam (d.h., als Acrylsäurebegleiter) mit der Acrylsäure in die kondensierte Phase überführt).

Demgemäß beansprucht die vorliegende Anmeldung ein Verfahren der heterogen katalysierten partiellen Gasphasenoxidation von Propylen zu Acrylsäure, bei dem man in einer ersten Reaktionszone ein Propylen, molekularen Sauerstoff und wenigstens ein inertes Verdünnungsgas enthaltendes Reaktionsgasausgangsgemisch 1, das den molekularen Sauerstoff und das Propylen in einem molaren Verhältnis O₂ : C₃H₆ ≥ 1 enthält, zunächst in einer ersten Reaktionsstufe (Reaktionsstufe 1) so durch wenigstens ein erstes Katalysatorbett (Katalysatorbett 1) führt, dessen Katalysatoren als Aktivmasse wenigstens ein Mo, Fe und Bi enthaltendes Multimetalloxid aufweisen, dass der Propylenumsatz bei einmaligen Durchgang durch das Katalysatorbett ≥ 90 mol-% und die damit einhergehende Selektivität S^{AC} der Acroleinbildung sowie der Acrylsäurenebenproduktbildung zusammen ≥ 80 (vorzugsweise ≥ 85, bzw. ≥ 90) mol-% betragen, die Temperatur des die erste Reaktionsstufe verlassenden Produktgasgemischs 1 durch direkte Kühlung, oder durch indirekte Kühlung, oder durch direkte und indirekte Kühlung gegebenenfalls verringert und dem Produktgasgemisch1 gegebenenfalls Sekundärgas in Form von molekularem Sauerstoff oder Inertgas, oder molekularem Sauerstoff und Inertgas zugibt, und danach das Produktgasgemisch 1 als Acrolein, molekularen Sauerstoff und wenigstens ein inertes Verdünnungsgas enthaltendes Reaktionsgasausgangsgemisch 2, das den molekularen Sauerstoff und das Acrolein in einem molaren Verhältnis O₂ : C₃H₄O ≥ 0,5 enthält, in einer zweiten Reaktionsstufe (Reaktionsstufe 2) bei erhöhter Temperatur so durch wenigstens ein zweites Katalysatorbett (Katalysatorbett 2) führt, dessen Katalysatoren als Aktivmasse wenigstens ein Mo und V enthaltendes Multimetalloxid aufweisen, dass der Acroleinumsatz bei einmaligem Durchgang durch das Kátalysatorbett ≥ 90 mol-% und die Selektivität S^{AA} der über beide Reaktionsstufen bilanzierten Acrylsäurebildung, bezogen auf umgesetztes Propylen, ≥ 70 (vorzugsweise ≥ 75, bzw. ≥ 80) mol-% beträgt, anschließend aus dem in der zweiten Reaktionsstufe gebildeten Produktgasgemisch 2 die darin enthaltene Acrylsäure in einer ersten Trennzone (Trennzone 1) in die kondensierte Phase überführt und danach in einer zweiten Trennzone (Trennzone 2) die Acrylsäure aus der kondensierten Phase durch Anwendung wenigstens eines thermischen Trennverfahrens abtrennt,
das dadurch gekennzeichnet ist,

dass das Reaktionsgasausgangsgemisch 1, bezogen auf die darin enthaltene molare Menge an Propylen, von > 0 bis zu 3 mol-% Cyclopropan enthält und das wenigstens eine thermische Trennverfahren in der zweiten Trennzone wenigstens eine kristallisative Abtrennung von Acrylsäure umfasst (aus einer kondensierten flüssigen Phase).

Unter kristallisativer Abtrennung von Acrylsäure wird dabei verstanden, dass sich die Acrylsäure im gebildeten Kristallisat und die Nebenkomponenten in der verbliebenen Mutterlauge anreichern.

Damit ist das erfindungsgemäße Verfahren insbesondere auch dann geeignet, wenn das Reaktionsgasausgangsgemisch 1, bezogen auf die darin enthaltene molare Menge an Propylen, 10 molppb bis 3 mol-%, oder 50 molppb bis 2 mol-%, oder 100 molppb bis 1 mol-%, oder 1 molppm bis 8000 molppm, oder 10 molppm bis 5000 molppm, oder 100 molppm bis 3000 molppm, oder 200 molppm bis 2500 molppm, oder 300 molppm bis 2000 molppm, oder 400 molppm, bzw. 500 molppm bis 1500 molppm, oder 750 bis 1250 molppm an Cyclopropan enthält.

Als Verfahren zur Überführung von im Produktgasgemisch 2 enthaltener Acrylsäure in die kondensierte Phase (die dabei in der Regel verbleibende Gasphase wird in dieser Schrift als Restgas bezeichnet) kommen in der Trennzone 1 beim erfindungsgemäßen Verfahren prinzipiell alle im Stand der Technik diesbezüglich bekannten Verfahren in Betracht. Sie sind im wesentlichen dadurch gekennzeichnet, dass man das Zielprodukt (die Acrylsäure) durch absorptive und/oder kondensative (Abkühlen) Maßnahmen aus der gasförmigen in die kondensierte Phase überführt.

Als Absorptionsmittel kommen dabei z. B. Wasser, wässrige Lösung und/oder organische (insbesondere hydrophobe) Lösungsmittel in Betracht (vgl. DE-A 103 36 386, DE-A 196 31 645, DE-A 195 01 325, EP-A 982 289, DE-A 198 38 845, WO 02/076917, EP-A 695 736, EP-A 778 225, EP-A 1 041 062, EP-A 982 287, EP-A 982 288, US 2004/0242826, EP-A 792 867, EP-A 784 046, EP-A 695 736 und in die diesen Schriften diesbezüglich zitierte Literatur).

Die im Produktgasgemisch 2 enthaltene Acrylsäure kann aber auch durch vollständige oder auch durch fraktionierende Kondensation in die kondensierte Phase überführt werden (z. B. WO 04/035514, DE-A 199 24 532, DE-A 198 14 387, DE-A 197 40 253, DE-A 197 40 252, DE-A 196 27 847 und die in diesen Schriften diesbezüglich zitierte Literatur).

Sowohl die absorptive als auch die kondensative Überführung der Acrylsäure in die flüssige Phase werden üblicherweise in trennwirksame Einbauten (zur Vergrößerung der Austauschoberfläche) enthaltenden Trennkolonnen vorgenommen. Als trennwirksame Einbauten kommen dabei alle bekannten Einbauten in Betracht. D. h., sowohl Böden wie Glockenböden, Dual-Flow-Böden, oder Ventilböden, Füllkörper wie z. B. Raschig-Ringe, oder Packungen, wie z. B. Sulzer-Packungen, können als trennwirksame Einbauten verwendet werden. In die Trennkolonne wird das Produktgasgemisch 2 dabei in der Regel von unten nach oben aufsteigend geführt. Im Rahmen einer absorptiven Kondensation wird in der Trennkolonne normalerweise von oben nach unten das Absorptionsmittel bewegt (geführt). Das flüssig ablaufende Absorbat bildet die die Acrylsäure (und schwerer sowie ähnlich wie diese siedende Nebenkomponenten wie Propionsäure) enthaltende kondensierte Phase. Bei der fraktionierenden Kondensation erfolgt die Kondensation der schwerer flüchtigen Bestandteile des Produktgasgemischs 2 in sich selbst aufsteigend. Das die Acrylsäure angereichert enthaltende Kondensat wird in der Regel über Seitenabzug aus der Kondensationskolonne herausgeführt. Selbstredend können Absorption und Kondensation auch einander überlagernd angewendet werden. Dies ist z. B. immer dann gegeben, wenn man dem System beim Absorptionsprozess durch direkte und/oder indirekte Kühlung zusätzlich Wärme entzieht. Vorzugsweise führt man das Produktgasgemisch 2 mit einer durch indirekte Kühlung, oder durch direkte Kühlung oder durch direkte und indirekte Kühlung verringerten Temperatur in die Trennkolonne. Die indirekte Kühlung wird dabei in an sich bekannter Weise in indirekten Wärmeaustauschern vorgenommen, während zur direkten Kühlung üblicherweise in einer Quenchvorrichtung vorgekühltes Absorptionsmittel oder vorgekühlte Sumpfflüssigkeit aus der Trennkolonne in das Produktgasgemisch 2 versprüht wird. Gemeinsames Merkmal der vorbeschriebenen absorptiven und/oder kondensativen Verfahren (Trennverfahren) ist, dass am Kopf der jeweiligen trennwirksame Einbauten enthaltenden Trennkolonne, in deren unteren Teil das Produktgasgemisch 2, wie beschrieben zweckmäßig nach vorheriger direkter und/oder indirekter Kühlung desselben, üblicherweise zugeführt wird, normalerweise ein Restgasstrom verbleibt, der hauptsächlich diejenigen Bestandteile des Produktgasgemischs 2 enthält, deren Siedepunkt bei Normaldruck (1 bar) ≤ -20°C beträgt (d. h., die schwer kondensierbaren oder auch leichter flüchtigen Bestandteile).

Dazu zählen z. B. als inertes Verdünnungsgas in der Partialoxidation mitverwendeter molekularer Stickstoff, in der Partialoxidation relativ zur Reaktionsstöchiometrie verbliebener überschüssiger molekularer Sauerstoff, als Nebenprodukte gebildete oder als inerte Verdünnungsgase im Reaktionsgasausgangsgemisch 1 mitverwendete Kohlenoxide, aber auch in der Partialoxidation nicht umgesetztes Propylen und nicht umgesetztes Cyclopropan. In der Regel wird das verbliebene Restgas z. B. auch noch Anteile von Wasserdampf enthalten. Erfindungsgemäß zweckmäßig wird man wenigstens eine Teilmenge eines solchen Restgases als Bestandteil des Reaktionsgasausgangsgemischs 1 in die Partialoxidation rückführen. Eine solche Kreisgasführung kann anwendungstechnisch zweckmäßig auch über eine der erfindungsgemäßen Partialoxidation als Propylenquelle vorgeschaltete heterogen katalysierte partielle Dehydrierung und/oder Oxidehydrierung von Propan erfolgen. Häufig wird man beim erfindungsgemäßen Verfahren wenigstens 10 Vol.-%, oder wenigstens 20 Vol.-%, oder wenigstens 30 Vol.%, meist jedoch nicht mehr als 80 Vol.-%, bzw. nicht mehr als 60 Vol.-%, bzw. nicht mehr als 40 Vol.-% des Restgases in die Partialoxidation rückführen (in der Regel jedoch möglichst vollständig, die darin enthaltene Gesamtmenge an nicht umgesetztem Propan und/oder Propen und mit diesen an nicht umgesetztem Cyclopropan). Ein Teil dieser Rückführung kann auch in die zweite Reaktionsstufe hinein erfolgen, d. h., als Bestandteil des Reaktionsgasausgangsgemischs 2.

Eine wie beschrieben durchgeführte Kreisgasführung kann einerseits als Inertgasquelle fungieren und erhöht in der Regel die angestrebte Zielproduktausbeute (bezogen auf eingesetzte Rohstoffmenge). Prinzipiell kann aber auch die Gesamtmenge und/oder eine Teilmenge des Restgases wie z. B. in der EP-A 925 272 beschrieben, der Verbrennung zugeführt werden (z. B. zur Energieerzeugung).

Absorptive und/oder kondensative Abtrennungen von Acrylsäure aus Produktgasgemischen 2 finden sich auch beschrieben in den Schriften EP-A 1 388 533,
EP-A 1 388 532, DE-A 102 35 847, WO 98/01415, EP-A 1 015 411, EP-A 1 015 410,
WO 99/50219, WO 00/53560, WO 02/09839, DE-A 102 35 847, WO 03/041833,
DE-A 102 23 058, DE-A 102 43 625, DE-A 103 36 386, EP-A 854 129,
US-A 4,317,926, DE-A 198 37 520, DE-A 196 06 877, DE-A 195 01 325,
DE-A 102 47 240, DE-A 197 40 253, EP-A 695 736, EP-A 1 041 062, EP-A 117 146,
DE-A 43 08 087, DE-A 43 35 172, DE-A 44 36 243, DE-A 103 32 758 sowie
DE-A 199 24 533.

Eine absorptive und/oder kondensative Abtrennung der Acrylsäure aus dem Produktgasgemisch 2 kann aber auch wie in der DE-A 103 36 386, der DE-A 101 15 277, DE-A 196 06 877, EP-A 920 408, EP-A 1 068 174, der EP-A 1 066 239, der EP-A 1 066 240, der WO 00/53560, der WO 00/53561, der DE-A 100 53 086, der WO 01/96271 bzw. wie in der DE-A 10 2004 032 129 und deren äquivalenten Schutzrechten beschrieben durchgeführt werden. Günstige Abtrennweisen sind auch die in den Schriften WO 04/063138, WO 04/35514, DE-A 102 43 625 und DE-A 102 35 847 beschriebenen Verfahren. Grundsätzlich kann die Acrylsäure aus dem Produktgasgemisch 2 in der ersten Trennzone auch ausgefroren werden.

Die weitere Abtrennung der Acrylsäure aus der kondensierten Phase kann nun beim erfindungsgemäßen Verfahren je nach in der Trennzone 1 angewandter Verfahrensweise, sowie je nach den im einzelnen für die Partialoxidation gewählten und damit das sonstige Nebenkomponentenspektrum bestimmenden Verfahrensbedingungen (Reaktionstemperatur, gewählte inerte Verdünnungsgase, gewählte Katalysatoren, Gehalt an und molares Verhältnis der Reaktanden im Reaktionsgasausgangsgemisch 1 etc.) durch unterschiedlichste Kombinationen unterschiedlichster thermischer Trennverfahren bis zum gewünschten Reinheitsgrad der Acrylsäure vorgenommen werden. Dies können z. B. Kombinationen von extraktiven, desorptiven, rektifikativen, azeotropdestillativen, azeotrop-rektifikativen, destillativen und/oder Strippverfahren sein.

Erfindungsgemäße wesentlich ist lediglich, dass die Kombination der insgesamt zur weiteren Abtrennung der Acrylsäure in der Trennzone 2 angewandten thermischen Trennverfahren wenigstens eine kristallisative Abtrennung von Acrylsäure umfasst. Dieses Erfordernis wird dadurch bedingt, dass mit einer kristallisativen Abtrennung von Acrylsäure eine erhöhte Propionsäureabreicherung verbunden ist, wohingegen mit anderen thermischen Trennverfahren eine solche Abreicherung nicht zu erzielen ist. D.h., aus wenigstens einer im Rahmen des wenigstens einen in der Trennzone 2 anzuwendenden thermischen Trennverfahrens anfallenden flüssigen, Acrylsäure enthaltenden, Phase wird beim erfindungsgemäßen Verfahren durch Abkühlen Kristallisat abgeschieden, in welchem Acrylsäure angereichert enthalten ist. Im Normalfall wird das Kristallisat im wesentlichen weitgehend ausschließlich aus Acrylsäure bestehen. Prinzipiell kann wenigstens eine kristallisative Abtrennung der Acrylsäure beim erfindungsgemäßen Verfahren aus einer bei der Überführung der Acrylsäure aus dem Produktgasgemisch 2 in die kondensierte Phase anfallenden acrylsäurehaltigen kondensierten Phase heraus erfolgen.

Beispielsweise kann die kristallisative Abtrennung der Lehre der DE-A 198 38 845 folgend unmittelbar aus dem Acrylsäure absorbiert enthaltenden Absorbat heraus erfolgen (unter Umständen kann selbiges zuvor noch einer Strippung und/oder Desorption unterworfen werden, um aus dem Absorbat wesentlich leichter als Acrylsäure flüchtige Bestandteile vorab der Kristallisation abzutrennen; derartige leichter flüchtige Bestandteile können z. B. Essigsäure, Ameisensäure und/oder niedere Aldehyde sein). Gegebenenfalls kann vorab der kristallisativen Abtrennung auch noch Absorptionsmittel destillativ entfernt werden, um so den Gehalt an Acrylsäure im Absorbat zu erhöhen und dadurch sicher zu stellen, dass Acrylsäure die beim Abkühlen sich abscheidende kristalline Phase (bzw. die Acrylsäure angereichert enthaltende sich abscheidende kristalline Phase) bildet. Das Vorgenannte ist sowohl dann zutreffend, wenn das Absorbat Acrylsäure und ein höher als Acrylsäure siedendes organisches Lösungsmittel enthält (vgl. z. B. DE-A 198 38 845; mögliche Absorptionsmittel sind z. B. Diphenyl, Diphenylether, Mischungen der beiden vorgenannten Mittel sowie Gemische aus Diphenyl, Diphenylether und Dimethylphthalat), als auch wenn als Absorptionsmittel Wasser oder eine wässrige Lösung verwendet wurde (vgl. z. B. WO 02/055469 und WO 03/078378). In der Regel wird man das im Rahmen der kristallisativen Abtrennung anfallende Kristallisat als weitere Trenn- bzw. Reinigungsmaßnahme zusätzlich noch waschen. Als Waschmittel kommt z. B. reines Absorptionsmittel und/oder die gewünschte Reinheit bereits aufweisende und vorab hergestellte Acrylsäure in Betracht. Prinzipiell kann ein Waschen auch durch Schwitzen erfolgen. Dabei wird das Kristallisat erwärmt und die bei tieferer Temperatur schmelzenden, erhöht verunreinigten Kristalle werden flüssig und fließen mit den Verunreinigungen als Waschflüssigkeit ab. Die Kristallisation kann aber auch unmittelbar aus dem im Rahmen einer fraktionierenden Kondensation anfallenden (vorzugsweise über Seitenabzug entnommenen), die Acrylsäure angereichert enthaltenden Kondensat heraus erfolgen, wie es z. B. die WO 04/035514 beschreibt. Grundsätzlich kann das Waschen des abgeschiedenen Kristallisats auch in einer Waschkolonne vorgenommen werden (dies kann eine statische, eine hydraulische oder eine mechanische Waschkolonne sein, wie es z. B. die WO 01/77056 beschreibt).

Grundsätzlich unterliegt die erfindungsgemäß anzuwendende kristallisative Behandlung wenigstens einer Acrylsäure enthaltenden flüssigen Phase P in der Trennzone 2 keiner Beschränkung, einschließlich den Verfahren zur Abtrennung der Mutterlauge vom Kristallisat (alle im in dieser Schrift genannten Stand der Technik ausgeführten Verfahren sind anwendbar). D. h., sie kann einstufig, oder mehrstufig, kontinuierlich oder diskontinuierlich durchgeführt werden. Insbesondere kann sie auch als fraktionierte (bzw. fraktionierende) Kristallisation durchgeführt werden. Üblicherweise werden bei einer fraktionierten Kristallistion alle Stufen, die ein Acrylsäurekristallisat erzeugen, das reiner als die zugeführte flüssige Phase P ist, Reinigungsstufen genannt und alle anderen Stufen Abtriebsstufen. Zweckmäßig werden mehrstufige Verfahren nach dem Gegenstromprinzip betrieben, bei dem nach der Kristallisation in jeder Stufe das Kristallisat von der Mutterlauge abgetrennt und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Häufig liegt die Temperatur der flüssigen Phase P beim Abscheiden der die Acrylsäure angereichert enthaltenden Kristalle zwischen -25°C und +14°C, insbesondere zwischen 12°C und -5°C.

Beispielsweise kann die erfindungsgemäß erforderliche kristallisative Abtrennung der Acrylsäure aus der diese enthaltende flüssigen Phase P als Schichtkristallisation ausgeführt sein (vgl. DE-A 26 06 364, EP-A 616 998, EP-A 648 520 und EP-A 776 875). Dabei wird das Kristallisat in Form zusammenhängender, fest anhaftender Schichten ausgefroren. Die Trennung des abgeschiedenen Kristallisats von der verbliebenen Restschmelze (auch Mutterlauge genannt) erfolgt im einfachsten Fall durch einfaches Abfließen der Restschmelze. Prinzipiell unterscheidet man zwischen "statischen" und "dynamischen" Schichtkristallisationsverfahren. Kennzeichnend für die dynamische Schichtkristallisation von flüssigen, Acrylsäure enthaltenden Phasen P ist eine erzwungene Konvektion der flüssigen Phase P. Diese kann durch Umpumpen der flüssigen Phase P durch volldurchströmte Rohre, durch Aufgabe der flüssigen Phase P als Rieselfilm (z. B. gemäß EP-A 616 998) oder durch Einleiten von Inertgas in eine flüssige Phase P oder durch Pulsieren erfolgen.

Bei den statischen Verfahren ruht die flüssige Phase P (z. B. in Rohrbündel- oder Plattenwärmetauschern) und scheidet sich schichtförmig durch langsame Temperatursenkung auf der Sekundärseite ab. Danach wird die Restschmelze (Mutterlauge) abgelassen, durch langsame Temperaturerhöhung stärker verunreinigte Fraktionen aus der Kristallschicht abgeschwitzt und nachfolgend das Reinprodukt abgeschmolzen (vgl. WO 01/77056).

Häufig wird man zur wenigstens einen kristallisativen Abtrennung der Acrylsäure in der Trennzone 2 eine Kombination von dynamischer und statischer Schichtkristallisation anwenden (vgl. EP-A 616 998).

Erfindungsgemäß bevorzugt wird die wenigstens eine kristallisative Abtrennung der Acrylsäure aus einer flüssigen Phase P (insbesondere aus allen in dieser Schrift beispielhaften ausgeführten flüssigen Phasen P) jedoch gemäß der Lehre der WO 01/77056, der WO 02/055469 und WO 03/078378 als Suspensionskristallisation ausgeführt.

Dabei wird in der Regel durch Kühlung der flüssigen Phase P eine Acrylsäurekristalle suspendiert enthaltende Kristallsuspension erzeugt, wobei die Acrylsäurekristalle einen geringeren und die verbleibende Restschmelze (Mutterlauge) einen höheren Propionsäuregehalt (relativ bezogen auf die jeweilige Gesamtmenge) aufweist, als die flüssige Phase P.

Dabei können die Acrylsäurekristalle unmittelbar in Suspension befindlich wachsen und/oder sich als Schicht auf einer gekühlten Wand abscheiden, von der sie anschließend abgekratzt und in der Restschmelze (Mutterlauge) resuspendiert werden.

Alle in der WO 01/77056, WO 02/055469 sowie WO 03/078378 aufgeführten Suspensionskristalle und Suspensionskristallisationsverfahren kommen erfindungsgemäß in Betracht. In der Regel weist die dabei erzeugte Acrylsäurekristallisatsuspension einen Feststoffgehalt von 20 bis 40 Gew.-% auf.

Weiterhin kommen alle in den vorgenannten WO-Veröffentlichungen genannten Verfahren zur Trennung von gebildetem Suspensionskristallisat und verbliebener Mutterlauge in Betracht (z.B. mechanische Trennverfahren wie Zentrifugieren). Erfindungsgemäß bevorzugt erfolgt die Trennung in einer Waschkolonne (z. B. eine gravimetrische, hydraulische oder mechanische; vgl. WO 01/77056). Bevorzugt handelt es sich dabei um eine Waschkolonne mit erzwungenem Transport der abgeschiedenen Acrylsäurekristalle. Der Kristallvolumenanteil im Kristallbett erreicht dabei in der Regel Werte > 0,5. Im Regelfall wird die Waschkolonne bei Werten von 0,6 bis 0,75 betrieben. Als Waschflüssigkeit wird mit Vorteil die Schmelze von in der Waschkolonne vorab gereinigten (abgetrennten) Acrylsäurekristallen verwendet. Die Wäsche erfolgt normalerweise im Gegenstrom. Damit umfasst das erfindungsgemäße Verfahren insbesondere Verfahren, welche folgende Verfahrensschritte umfassen:
a) Auskristallisieren von Acrylsäure aus einer flüssigen Phase P,
b) Trennen des Acrylsäurekristallisats von der verbliebenen Mutterlauge (Restschmelze, flüssigen Restphase),
c) wenigstens teilweises Aufschmelzen des abgetrennten Acrylsäurekristallisats und
d) wenigstens teilweises Rückführen des aufgeschmolzenen Acrylsäurekristallisats zum Schritt b) und/oder zum Schritt a).

Bevorzugt erfolgt dabei der Schritt b) durch Gegenstromwäsche mit in den Schritt b) rückgeführtem aufgeschmolzenem zuvor abgetrenntem Acrylsäurekristallisat. Erfindungsgemäß vorteilhaft enthält die flüssige Phase P bei Anwendung der erfindungsgemäßen kristallisativen Acrylsäureabtrennung Wasser, da eine Ausbildung von Acrylsäurekristallisat im Beisein von Wasser gemäß der Lehre der WO 01/77056 und WO 03/078378 eine für die nachfolgende Trennung des Kristallisats von verbliebener Mutterlauge für das erfindungsgemäße Vorhaben besonders günstige Kristallform bedingt. Dies gilt insbesondere dann, wenn die Kristallisation als Suspenskristallisation ausgeführt wird, und noch mehr, wenn die nachfolgende Mutterlaugeabtrennung in einer Waschkolonne ausgeführt wird, und noch mehr, wenn dabei als Waschflüssigkeit die Schmelze von in der Waschkolonne bereits gereinigtem Acrylsäurekristallisat verwendet wird.

D.h., die erfindungsgemäß erforderliche kristallisative Abtrennung von Acrylsäure umfasst insbesondere Verfahren, bei denen man die Acrylsäure enthaltende flüssige Phase P unter Einwirkung von Kälte in eine aus Acrylsäurekristallisat und flüssiger Restphase (Restschmelze) bestehende Kristallisatsuspension überführt, wobei der Gewichtsanteil des Acrylsäurekristallisats an Propionsäure kleiner und der Gewichtsanteil der flüssigen Restphase (der Mutterlauge) an Propionsäure größer als der Propionsäuregewichtsanteil der flüssigen Phase P ist, von der Kristallisatsuspension gegebenenfalls einen Teil der verbliebenen Mutterlauge mechanisch abtrennt und das Acrylsäurekristallisat in einer Waschkolonne von verbliebener Mutterlauge mit der Maßgabe befreit, dass
a) die flüssige Phase P bezogen auf die darin enthaltene Acrylsäure 0,20 bis 30, häufig bis 20, oft bis 10 Gew.-% an Wasser enthält, und
b) als Waschflüssigkeit die Schmelze von in der Waschkolonne gereinigtem Acrylsäurekristallisat verwendet wird.

Insbesondere umfasst das erfindungsgemäße Verfahren vorstehende Verfahren, wobei die flüssige Phase P ≥ 80 Gew.-% Acrylsäure, oder ≥ 90 Gew.-% Acrylsäure, oder 95 Gew.-% Acrylsäure aufweist.

Weiterhin ist es erfindungsgemäß vorteilhaft, wenn der Wassergehalt der flüssigen Phase P bei vorstehend beschriebenen Verfahrensweisen (bzw. ganz generell bei Anwendung des erfindungsgemäßen Verfahrens), bezogen auf in der flüssigen Phase P enthaltene Acrylsäure, 0,2 bzw. 0,4 bis 8, bzw. bis 10, bzw. bis 20, bzw. bis 30 Gew.-%, bzw. 0,6 bis 5 Gew.-%, bzw. 0,60 bis 3 Gew.-% beträgt.

Alles Vorgenannte gilt vor allem dann, wenn die Waschkolonne eine Waschkolonne mit erzwungenem Transport der Acrylsäurekristalle ist, und dies vor allem dann, wenn es eine hydraulische oder eine mechanische Waschkolonne gemäß der WO 01/77056 ist und sie wie dort ausgeführt betrieben wird.

Alles Vorgenannte gilt vor allem dann, wenn die Waschkolonne gemäß den Lehren der WO 03/041832 sowie der WO 03/041833 ausgeführt ist und betrieben wird.

Die wenigstens eine kristallisative Abtrennung von Acrylsäure muss erfindungsgemäß nicht in notwendiger Weise unmittelbar aus der in der Trennzone 1 anfallenden, die Acrylsäure in kondensierter Form enthaltenden kondensierten Phase heraus erfolgen. Vielmehr wird man die in der Trennzone 1 gewonnene, die Acrylsäure enthaltende flüssige (kondensierte) Phase zunächst einem oder auch einer Kombination von thermischen Trennverfahren unterwerfen, im Rahmen derer die erfindungsgemäß zu kristallisierende acrylsäurehaltige flüssige Phase P anfällt.

Vielfach wird die erfindungsgemäß zu kristallisierende acrylsäurehaltige flüssige Phase P somit das Ergebnis einer Anwendung von wenigstens einem von einer Kristallisation verschiedenen thermischen Trennverfahren (z. B. Destillation, Rektifikation, Extraktion, Destraktion, Desoprtion, Strippung, azeotroper Rektifikation, Adsorption und/oder azeotroper Destillation) auf die in der Trennzone 1 gewonnene acrylsäurehaltige kondensierte (flüssige) Phase und/oder auf im Rahmen dieser Anwendung resultierende Folgephasen sein (vgl. z. B. DE-A 196 06 877). Häufig wird man zur Erzeugung der kristallisativ zu behandelnden acrylsäurehaltigen flüssigen Phase P vorgenannte Verfahren mehrfach anwenden. Beispielsweise kann die erfindungsgemäß zu kristallisierende acrylsäurehaltige flüssige Phase P eine wie in der DE-A 103 36 386 aus dem in der Trennzone 1 anfallenden Absorbat des Produktgasgemischs der Propylen-Partialoxdation durch Anwendung verschiedener von einer Kristallisation verschiedenen thermischen Trennverfahren gewonnene rohe Acrylsäure sein. Eine solche erfindungsgemäß zu kristallisierende rohe Acrylsäure kann aber auch aus einem in der Trennzone 1 des erfindungsgemäßen Verfahrens anfallenden wässrigen Absorbat gemäß der Lehren der EP-A 695 736, EP-A 778 255 bzw. EP-A 1 041 062 unter Mitanwendung wenigstens einer azeotropen Destillation gewonnen werden.

Der Propionsäureauslass kann sich beim erfindungsgemäßen Verfahren grundsätzlich ausschließlich in der wenigstens einen kristallisativen Acrylsäureabtrennung befinden. In diesem Fall wird der Auslass aus. Propionsäure angereichert enthaltender Mutterlauge bestehen.

Wird die erfindungsgemäß anzuwendende kristallisative Abtrennung z. B. mittels einer Kombination von dynamischer und statischer Kristallisation gemäß der EP-A 616 998 ausgeführt, wird sich der Propionsäure angereichert enthaltende Mutterlaugeauslass zweckmäßig im Bereich der statischen Kristallisation befinden.

Erfindungsgemäß vorteilhaft wird man beim erfindungsgemäßen Verfahren das scharfe Trennverfahren der wenigstens einen kristallisativen Acrylsäureabtrennung in der zweiten Trennzone mit wenigstens einem unscharfen Trennverfahren in der ersten (bevorzugt) und/oder zweiten Trennzone dadurch rückkoppeln, dass man die bei der kristallisativen Acrylsäureabtrennung verbleibende Mutterlauge wenigstens teilweise in wenigstens eines der unscharfen Trennverfahren rückführt.

Die Grundstruktur einer solchermaßen gekoppelten Anwendung von unscharfen Trennverfahren und dem scharfen Trennverfahren der Kristallisation lehren z. B. die DE-A 196 06 877, die EP-A 792 867 sowie die EP-A 1 484 308, die EP-A 1 484 309, die EP-A 1 116 709 und insbesondere die EP-A 1 015 410.

Ein unscharfes Trennverfahren ist dabei als ein Trennverfahren definiert, bei dem die Zusammensetzung der sich bei Anwendung des Trennverfahrens bildenden, Zielprodükt angereichert enthaltenden, Phase von der Zusammensetzung des zu trennenden Gemischs in ausgeprägter Weise abhängig ist, während die erfindungsgemäß erforderliche kristallisative Abtrennung insofern ein scharfes Trennverfahren ist, als die Zusammensetzung der sich bildenden Acrylsäurekristalle weitgehend unabhängig (im Idealfall besteht völlige Unabhängigkeit) von der Zusammensetzung der acrylsäurehaltigen flüssigen Phase P ist. Eine in der Trennzone 1 angewandte Absorption und/oder fraktionierende Kondensation zur Überführung von Acrylsäure aus dem Produktgasgemisch 2 in die kondensierte Phase sind ebenso unscharfe Trennverfahren wie z. B. eine Extraktion und/oder Rektifikation in der Trennzone 2.

Das erfindungsgemäße Verfahren ist im Fall einer solchen Kopplung eines scharfen Trennverfahrens in der Trennzone 2 und eines unscharfen Trennverfahrens in z. B. der Trennzone 1 insofern von erhöhter Bedeutung, als sich beim kontinuierlichen Betrieb einer solchen Verfahrensweise die Propionsäure in der erfindungsgemäß kristallisativ zu behandelnden acrylsäurehaltigen flüssigen Phase P durch die Mutterlaugerückführung aufpegelt, da die Mutterlauge die Propionsäure angereichert enthält.

In zweckmäßiger Weise weist eine wie beschrieben anzuwendende Kopplung von unscharfer und scharfer Trennung im Rahmen des erfindungsgemäßen Verfahrens auch einen Auslass (dies kann der einzige Auslass des Verfahrens sein) für wenigstens einen Propionsäure angereichert enthaltenden Stoffstrom jenseits der Mutterlauge auf. Beispielsweise kann man als solchen Auslass die Sumpfflüssigkeit einer Trennkolonne (z. B. der Absorptions- oder Kondensationskolonne in der Trennzone 1) verwenden, aus der die flüssige Phase P selbst oder der im weiteren Verlauf in die flüssige Phase P zu wandelnde Stoffstrom z. B. über Seitenentnahme und/oder Kopfentnahme entnommen wird.

Alternativ kann man aber auch an einer Stelle in der Trennkolonne (z. B. der in der Trennzone 1 befindlichen), an der sich ein Propionsäurebauch befindet (zusätzlich zum Auslass für die flüssige Phase P), einen separaten Propionsäureauslassstrom entnehmen und aus diesem in einer zweiten, vorzugsweise fraktionierten krisallisativen Abtrennung (z. B. Kombination aus dynamischer und statischer Kristallisation gemäß EP-A 616 998) die Propionsäure in der Mutterlauge anreichern und solche Mutterlauge (vorzugsweise aus der statischen Kristallisation) auslassen. Von der aufgereinigten Kristallfraktion kann aufgeschmolzen in die Trennkolonne rückgeführt und/oder in die Kristallisation der acrylsäurehaltigen flüssigen Phase P eingeschleust werden. Prinzipiell kann ein solcher Propionsäureauslassstrom auch ein der flüssigen Phase P entnommener Teilstrom sein.

Selbstverständlich werden alle in den Trennzonen 1, 2 ausgeführten Verfahrensschritte polymerisationsinhibiert durchgeführt. Dabei kann wie im aufgeführten Stand der Technik beschrieben vorgegangen werden. Eine herausragende Position unter der Gesamtmenge der verfügbaren Acrylsäure-Prozessstabilisatoren nehmen Dibenzo-1,4-thiazin (PTZ), 4-Hydroxy-2,2,6,6-tetramethylpiperidin-1-oxyl (4-OH-TEMPO) und p-Methoxyphenol (MEHQ) ein. Sie können z. B. entweder jeweils für sich, oder paarweise oder als Dreiergemisch Bestandteil der erfindungsgemäß kristallisativ zu behandelnden acrylsäurehaltigen flüssigen Phase P sein. Üblicherweise beträgt die Gesamtmenge von an in der flüssigen Phase P enthaltenen Polymerisationsinhibitoren, bezogen auf darin enthaltene Acrylsäure, 0,001 bis 2 Gew.-%.

Insgesamt gestattet es das erfindungsgemäße Verfahren trotz des Gehalts an Cyclopropan im Reaktionsgasausgangsgemisch 1 mit der Sequenz zweistufige Partialoxidatiön des im Reaktionsgasausgangsgemisch 1 enthaltenen Propylen, fraktionierende Acrylsäurekondensation aus dem Produktgasgemisch 2 der Partialoxidation, Suspensionskristallisation des entnommenen Acrylsäurekondensats und Abtrennung des Suspensionskristallisats von verbliebener Mutterlauge in einer Waschkolonne unter Anwendung einer Reinkristallisatschmelze als Waschflüssigkeit, auf effiziente Art und Weise und unter Anwendung lediglich einer Kristallisationsstufe die Herstellung von superabsorbertauglicher Acrylsäure (solche Acrylsäure kann selbstredend auch für alle anderen in der WO 02/055469 und WO 03/078378 angesprochenen Verwendungen eingesetzt werden.

Abgesehen vom Cyclopropangehalt des Reaktionsgasausgangsgemischs 1 ist das eingangs dieser Schrift umrissene Verfahren der zweistufigen heterogen katalysierten partiellen Gasphasenoxidation von Propylen zu Acrylsäure allgemein bekannt (vgl. z. B. WO 01/36364). Bei den Katalysatorbetten kann es sich dabei um Festbetten oder um Wirbelbetten handeln. Erfindungsgemäß bevorzugt ist in beiden Reaktionsstufen die Verwendung von Katalysatorfestbetten.

Unter der Belastung des Katalysator(fest)betts mit Reaktionsgasausgangsgemisch wird in dieser Schrift die Menge an Reaktionsgasausgangsgemisch in Normlitern (= NI; das Volumen in Litern, das die entsprechende Menge Reaktionsgasausgangsgemisch bei Normalbedingungen, d.h., bei 25°C und 1 bar, einnehmen würde) verstanden, die pro Stunde durch einen Liter Katalysator(fest)bett geführt wird. Die Belastung des Katalysator(fest)betts kann auch nur auf eine Komponente des Reaktionsgasausgangsgemischs bezogen sein. Dann ist sie die Menge dieser Komponente in Normlitern, die als Bestandteil des entsprechenden Reaktionsgasausgangsgemischs pro Stunde durch einen Liter des Katalysator(fest)betts geführt wird.

Die Realisierung der erfindungsgemäß durchzuführenden zweistufigen heterogen katalysierten Partialoxidation von Propylen zu Acrylsäure unter Verwendung eines erfindungsgemäßen Reaktionsgasausgangsgemischs 1 kann dabei im einzelnen z. B. wie in den Schriften EP-A 700 714 (erste Reaktionsstufe; wie dort beschrieben, aber auch in entsprechender Gegenstromfahrweise von Salzbad und Reaktionsgasausgangsgemisch über den Rohrbündelreaktor), EP-A 700 893 (zweite Reaktionsstufe; wie dort beschrieben, aber auch in entsprechender Gegenstromfahrweise), WO 04/085369 (insbesondere diese Schrift wird als integraler Bestandteil dieser Schrift betrachtet) (als zweistufiges Verfahren), WO 04/085363, DE-A 103 13 212 (erste Reaktionsstufe), EP-A 1 159 248 (als zweistufiges Verfahren), EP-A 1 159 246 (zweite Reaktionsstufe), EP-A 1 159 247 (als zweistufiges Verfahren), DE-A 199 48 248 (als zweistufiges Verfahren), DE-A 101 01 695 (zweistufig), WO 04/085368 (als zweistufiges Verfahren), DE-A 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), EP-A 990 636, EP-A 1 007 007 und EP-A 1 106 598 beschrieben durchgeführt werden.

Dies gilt insbesondere für alle in diesen Schriften enthaltenen Ausführungsbeispiele. Sie können wie in diesen Schriften beschrieben durchgeführt werden, jedoch mit dem Unterschied, dass als Reaktionsgasausgangsgemisch für die erste Reaktionsstufe ein erfindungsgemäßes Reaktionsgasausgangsgemisch 1 eingesetzt wird. Die übrigen Parameter betreffend kann wie in den Ausführungsbeispielen der genannten Schriften verfahren werden (insbesondere die Katalysatorfestbetten und die Reaktandenbelastung der Katalysatorfestbetten betreffend). Erfolgt beim erfindungsgemäßen Verfahren zwischen den beiden Reaktionsstufen eine Zufuhr von molekularem Sekundärsauerstoff, so erfolgt diese erfindungsgemäß vorzugsweise in Form von Luft. Sie kann aber auch als reiner molekularer Sauerstoff oder auch als ein sonstiges Gemisch aus molekularem Sauerstoff und aus Inertgas erfolgen. Erfindungsgemäß vorteilhaft erfolgt die Sekundärsauerstoffzufuhr in solcher Menge, dass das Produktgasgemisch 2 noch nicht umgesetzten molekularen Sauerstoff enthält. Allerdings kann die für das Gesamtverfahren benötigte Menge an molekularem Sauerstoff auch bereits dem Reaktionsgasausgangsgemisch 1 zugefügt werden. In der Regel wird das molare Verhältnis von im Reaktionsgasausgangsgemisch 1 enthaltenem molekularem Sauerstoff zu in diesem Gemisch enthaltenem Propylen ≥ 1 und ≤ 3 betragen.

Für die jeweilige der beiden Reaktionsstufen geeignete, die erfindungsgemäß geforderten Elemente enthaltende Multimetalloxidkatalysatoren sind vielfach vorbeschrieben und dem Fachmann wohlbekannt. Beispielsweise verweist die EP-A 253 409 auf Seite 5 auf entsprechende US-Patente. Geeignete Katalysatoren für die jeweilige Oxidationsstufe (Reaktionsstufe) offenbaren auch die DE-A 44 31 957, die DE-A 10 2004 025 445 und die DE-A 44 31 949. Dies gilt auch für jene der allgemeinen Formel I in den beiden vorgenannten älteren Schriften. Für die jeweilige Oxidationsstufe (Reaktionsstufe) verwendbare Katalysatoren offenbaren auch die Schriften DE-A 103 25 488, DE-A 103 25 487, DE-A 103 53 954, DE-A 103 44 149, DE-A 103 51 269, DE-A 103 50 812 und DE-A 103 50 822.

Für das erfindungsgemäße Verfahren in der ersten Reaktionsstufe mögliche, Mo, Fe und Bi enthaltende Multimetalloxidaktivmassen sind auch die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 7 00 714 genannten Multimetalloxidaktivmassen.

Ferner eignen sich für die erste Reaktionsstufe des erfindungsgemäßen Verfahrens die Mo, Bi und Fe enthaltenden Multimetalloxidkatalysatoren, die in den Schriften Research Disclosure Nr. 497012 vom 29.08.2005, DE-A 100 46 957, DE-A 100 63 162, DE-C 3 338 380, DE-A 199 02 562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften Ausführungsformen in diesen Schriften, unter denen jene der EP-A 15565, der EP-A 575897, der DE-A 197 46 210 und der DE-A 198 55 913 besonders bevorzugt werden. Besonders hervorzuheben sind in diesem Zusammenhang ein Katalysator gemäß Beispiel 1 c aus der EP-A 15 565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Oₓ·10SiO₂ aufweist. Ferner sind hervorzuheben das Beispiel mit der laufenden Nr. 3 aus der DE-A 198 55 913 (Stöchiometrie: MO₁₂Co₇Fe₃Bi_{0,6}K_{0,08}Si_{1,6}Oₓ) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl derjenigen Mo, Fe und Bi enthaltenden Multimetalloxidaktivmassen, in deren Beisein das Cyclopropan in der ersten Reaktionsstufe in besonderer Weise für die unerwünschte Nebenreaktion empfänglich und bei deren Verwendung die erfindungsgemäße Verfahrensweise daher besonders relevant ist, lässt sich unter der allgemeinen Formel IV,

Mo₁₂BiₐFe_{b}X¹_{c}X²_{d}X³ₑX⁴_{f}Oₙ , (IV)

in der die Variablen nachfolgende Bedeutung aufweisen:
- X¹: = Nickel und/oder Kobalt,
- X²: = Thallium, ein Alkalimetall und/oder ein Erdalkalimetall,
- X³: = Zink, Phosphor, Arsen, Bor, Antimon, Zinn, Cer, Blei und/oder Wolfram,
- X⁴: = Silicium, Aluminium, Titan und/oder Zirkonium,

- a: = 0,5 bis 5,
- b: = 0,01 bis 5, vorzugsweise 2 bis 4,
- c: = 0 bis 10, vorzugsweise 3 bis 10,
- d: = 0 bis 2, vorzugsweise 0,02 bis 2,
- e: = 0 bis 8, vorzugsweise 0 bis 5,
- f: = 0 bis 10 und
- n: = eine Zahl, die durch die Wertigkeit und Häufigkeit der von Sauerstoff verschiedenen Elemente in IV bestimmt wird,
subsumieren.

Das Vorgenannte gilt vor allem, wenn sie in an sich bekannter Weise erhalten (siehe z. B. die DE-A 4 023 239) und z. B. in Substanz zu Kugeln, Ringen oder Zylindern geformt oder auch in Gestalt von Schalenkatalysatoren, d. h., mit der Aktivmasse beschichteten vorgeformten, inerten Trägerkörpern, erfindungsgemäß eingesetzt werden. Selbstverständlich gilt das Gesagte aber auch, wenn sie in Pulverform als Katalysatoren für die erste Reaktionsstufe angewendet werden (z. B. in Wirbelbettreaktoren).

Prinzipiell werden Aktivmassen der allgemeinen Formel IV in einfacher Weise in der Regel dadurch hergestellt, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für die erste Reaktionsstufe des erfindungsgemäßen Verfahrens sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Anstelle von Graphit kann aber auch hexagonales Bornitrid als Hilfsmittel bei der Formgebung verwendet werden, wie es die DE-A 10 2005 037 678 empfiehlt. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine erfindungsgemäß besonders relevante Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der erfindungsgemäß relevanten pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird häufig im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig.geformte Trägerkörper mit deutlich ausgebildeter Oberflächenrauhigkeit, z.B. Kugeln oder Hohlzylinder, bevorzugt werden. Erfindungsgemäß relevant ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Erfindungsgemäß relevant ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Erfindungsgemäß zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß relevant sind auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein erfindungsgemäß relevante Multimetalloxidaktivmassen sind ferner Massen der allgemeinen Formel V,

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} , (V)

in der die Variablen folgende Bedeutung haben:
- Y¹: = nur Wismut oder Wismut und wenigstens eines der Elemente Tellur, Antimon, Zinn und Kupfer,
- Y²: = Molybdän, oder Wolfram, oder Molybdän und Wolfram,
- Y³: = ein Alkalimetall, Thallium und/oder Samarium,
- Y⁴: = ein Erdalkalimetall, Nickel, Kobalt, Kupfer, Mangan, Zink, Zinn, Cadmium und/oder Quecksilber,
- Y⁵: = Eisen oder Eisen und wenigstens eines der Elemente Chrom und Cer,
- Y⁶: = Phosphor, Arsen, Bor und/oder Antimon,
- Y⁷: = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

- a': = 0,01 bis 8,
- b': = 0,1 bis 30,
- c': = 0 bis 4,
- d': = 0 bis 20,
- e': > 0 bis 20,
- f': = 0 bis 6,
- g': = 0 bis 15,
- h': = 8 bis 16,
- x',y': = Zahlen, die durch die Wertigkeit und Häufigkeit der von Sauerstoff verschiedenen Elemente in V bestimmt werden und
- p,q: = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders relevante Multimetalloxidmassen V sind erfindungsgemäß solche, in denen Y¹ nur Wismut ist.

Unter diesen sind wiederum jene von besonderer Relevanz, die der allgemeinen Formel VI,

[Bi_{a''}Z²_{b''}O_{x''}]_{p''} [Z²₁₂Z³_{c''}Z⁴_{d''}Fe_{e''}Z⁵_{f''}Z⁶_{g''}Z⁷_{h''}O_{y''}]_{q''} , (VI)

in der die Variablen folgende Bedeutung haben:
- Z²: = Molybdän, oder Wolfram, oder Molybdän und Wolfram,
- Z³: = Nickel und/oder Kobalt,
- Z⁴: = Thallium, ein Alkalimetall und/oder ein Erdalkalimetall,
- Z⁵: = Phosphor, Arsen, Bor, Antimon, Zinn, Cer und/oder Blei,
- Z⁶: = Silicium, Aluminium, Titan und/oder Zirkonium,
- Z⁷: = Kupfer, Silber und/oder Gold,

- a": = 0,1 bis 1,
- b": = 0,2 bis 2,
- c": = 3 bis 10,
- d": = 0,02 bis 2,
- e": = 0,01 bis 5, vorzugsweise 0,1 bis 3,
- f": = 0 bis 5,
- g": = 0 bis 10,
- h": = 0 bis 1,
- x",y": = Zahlen, die durch die Wertigkeit und Häufigkeit der von Sauerstoff verschiedenen Element in VI bestimmt werden,
- p",q": =Zahlen, deren Verhältnis p"/q" 0,1 bis 5, vorzugsweise 0,5 bis 2 beträgt,
entsprechen, wobei diejenigen Massen VI ganz besonders bevorzugt werden, in denen Z²_{b''} = (Wolfram)_{b''} und Z²₁₂ = (Molybdän)₁₂ ist.

Ferner ist es erfindungsgemäß von Bedeutung, wenn wenigstens 25 mol-% (bevorzugt wenigstens 50 mol-% und besonders bevorzugt 100 mol-%) des gesamten Anteils [Y¹_{a'}Y²_{b'}O_{x'}]ₚ ([Bi_{a''}Z²_{b''}O_{x''}]_{p''}) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a''}Z²_{b''}O_{x''}] vorliegen, deren Größtdurchmesser im Bereich 1 nm bis 100 µm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.
Für den zweiten Schritt (die zweite Reaktionsstufe), die heterogen katalysierte Gasphasen-Partialoxidation von Acrolein zu Acrylsäure, kommen erfindungsgemäß prinzipiell alle Mo und V enthaltenden Multimetalloxidmassen als Aktivmassen für die benötigten Katalysatoren in Betracht, z. B. jene der DE-A 100 46 928 und der DE-A 198 15 281.

Eine Vielzahl derselben, die für die unerwünschte Reaktion des Cyclopropan erfindungsgemäß besonders relevant sind, lässt sich unter der allgemeinen Formel VII,

Mo₁₂VₐX¹_{b}X²_{c}X³_{d}X⁴ₑX⁵_{f}X⁶_{g}Oₙ , (VII),

in der die Variablen folgende Bedeutung haben:
- X¹: = W, Nb, Ta, Cr und/oder Ce,
- X²: = Cu, Ni, Co, Fe, Mn und/oder Zn,
- X³: = Sb und/oder Bi,
- X⁴: = eines oder mehrere Alkalimetalle,
- X⁵: = eines oder mehrere Erdalkalimetalle,
- X⁶: = Si, Al, Ti und/oder Zr,

- a: = 1 bis 6,
- b: = 0,2 bis 4,
- c: = 0,5 bis 18,
- d: = 0 bis 40,
- e: = 0 bis 2,
- f: = 0 bis 4,
- g: = 0 bis 40 und
- n: = eine Zahl, die durch die Wertigkeit und Häufigkeit der von Sauerstoff verschiedenen Elemente in VII bestimmt wird,
subsumieren.

Erfindungsgemäß besonders relevante Ausführungsformen innerhalb der aktiven Multimetalloxide VII sind jene, die von den nachfolgenden Bedeutungen der Variablen der allgemeinen Formel VII erfasst werden:
- X¹: = W, Nb, und/oder Cr,
- X²: = Cu, Ni, Co, und/oder Fe,
- X³: = Sb,
- X⁴: = Na und/oder K,
- X⁵: = Ca, Sr und/oder Ba,
- X⁶: = Si, Al, und/oder Ti,
- a: = 1,5 bis 5,
- b: = 0,5 bis 2,
- c: = 0,5 bis 3,
- d: = 0 bis 2,
- e: = 0 bis 0,2,
- f: = 0 bis 1 und
- n: = eine Zahl, die durch die Wertigkeit und Häufigkeit der von Sauerstoff verschiedenen Elemente in VII bestimmt wird. Erfindungsgemäß ganz besonders relevante Multimetalloxide VII sind jedoch jene der allgemeinen Formel VIII,

Mo₁₂V_{a'}Y¹_{b'}Y²_{c'}Y⁵_{f'}Y⁶_{g'}O_{n'}. , (VIII)

mit
- X¹: = W und/oder Nb,
- Y²: = Cu und/oder Ni,
- Y⁵: = Ca und/oder Sr,
- Y⁶: = Si und/oder Al,
- a': = 2 bis 4,
- b': = 1 bis 1,5,
- c': = 1 bis 3,
- f': = 0 bis 0,5
- g': = 0 bis 8 und
- n': = eine Zahl, die durch die Wertigkeit und Häufigkeit der von Sauerstoff verschiedenen Elementen in VIII bestimmt wird.

Die erfindungsgemäß relevanten Multimetalloxidaktivmassen (VII) sind in an sich bekannter, z. B. in der DE-A 43 35 973 oder in der EP-A 714 700 offenbarter, Weise erhältlich.

Generell können für den Schritt "Acrolein → Acrylsäure" erfindungsgemäß relevante Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für die erfindungsgemäße Acroleinoxidation sowohl in Pulverform (z. B. in Wirbelbettreaktoren) als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Relevante Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird in erfindungsgemäßer relevanter Weise häufig im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Relevant sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den erfindungsgemäßen Schritt "Acrolein → Acrylsäure" relevante Multimetalloxidaktivmassen sind ferner Massen der allgemeinen Formel IX,

[D]ₚ[E]_{q} , (IX)

in der die Variablen folgende Bedeutung haben:
- D: = Mo₁₂V_{a''}Z¹_{b''}Z²_{c''}Z²_{d''}Z⁴_{e''}Z⁵_{f''}Z⁶_{g''}O_{x''},
- E: = Z⁷₁₂Cu_{h''}H_{i''}O_{y''},
- Z¹: = W, Nb, Ta, Cr und/oder Ce,
- Z²: = Cu, Ni, Co, Fe, Mn und/oder Zn,
- Z³: = Sb und/oder Bi,
- Z⁴: = Li, Na, K, Rb, Cs und/oder H
- Z⁵: = Mg, Ca, Sr und/oder Ba,
- Z⁶: = Si, Al, Ti und/oder Zr,
- Z⁷: = Mo, W, V, Nb und/oder Ta, vorzugsweise Mo und/oder W
- a": = 1 bis 8,
- b": = 0,2 bis 5,
- c": = 0 bis 23,
- d": = 0 bis 50,
- e": = 0 bis 2,
- f": = 0 bis 5,
- g": = 0 bis 50,
- h": = 4 bis 30,
- i": = 0 bis 20 und
- x",y": = Zahlen, die durch die Wertigkeit und Häufigkeit der von Sauerstoff verschiedenen Element in IX bestimmt werden und
- p,q: = von Null verschiedene Zahlen, deren Verhältnis p/q 160:1 bis 1:1 beträgt,
und die dadurch erhältlich sind, dass man eine Multimetalloxidmasse E

Z⁷₁₂Cu_{h''}H_{i''}O_{y''} , (E)

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo₁₂V_{a''}Z¹_{b''}Z²_{c''}Z³_{d''}Z⁴_{e''}Z⁵_{f''}Z⁶_{g''} , (D)

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Besonders relevant sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" erfindungsgemäß besonders relevante Multimetalloxidkatalysatoren sind auch jene der DE-A 198 15 281, insbesondere mit Multimetalloxidaktivmassen der allgemeinen Formel I dieser Schrift.

Mit erfindungsgemäßer Relevanz werden für den Schritt vom Propylen zum Acrolein Vollkatalysatorringe und für den Schritt vom Acrolein zur Acrylsäure Schalenkatalysatorringe verwendet.

Die Reaktionstemperatur in der ersten Reaktionsstufe beträgt erfindungsgemäß zweckmäßig 270 bis 450°C bzw. 280 bis 420°C, vorzugsweise 300 bis 380°C. Die Reaktionstemperatur in der zweiten Reaktionsstufe beträgt erfindungsgemäß zweckmäßig 200 bis 370 bzw. bis 320°C, vorzugsweise 220 bis 300°C.

Ferner ist das erfindungsgemäße Verfahren von besonderer Relevanz, wenn die Aktivmassen für die Katalysatoren der ersten Reaktionsstufe solche umfassen, deren spezifische Oberfläche 0,1 bis 120 m²/g, bzw. 0,2 bis 50 m²/g, bzw. 1 bis 20 m²/g, oder 2 bis 10 m²/g beträgt.

Auch ist das erfindungsgemäße Verfahren von besonderer Relevanz, wenn die Aktivmassen für die Katalysatoren der ersten Reaktionsstufe solche umfassen, deren numerisch häufigster Porendurchmesser 0,1 bis 1 µm beträgt.

Es ist weiterhin von besonderer Relevanz, wenn bei den Aktivmassen für die Katalysatoren der ersten Reaktionsstufe vorgenannter numerisch häufigster Porendurchmesser und eine der vorgenannten spezifischen Oberflächen kombiniert vorliegen.

Außerdem ist das erfindungsgemäße Verfahren von besonderer Bedeutung, wenn bei den Aktivmassen für die Katalysatoren der ersten Reaktionsstufe der Anteil der verschiedenen Porendurchmesser am Porengesamtvolumen wie folgt verteilt ist:
Poren mit Durchmesser im Bereich < 0,03 µm : ≥ 0 und ≤ 5 Vol.-%.
Poren mit Durchmesser im Bereich ≥ 0,003 µm bis ≤ 0,1 µm : ≥ 3 und < 20 Vol.-%.

Poren mit Durchmesser im Bereich > 0,1 bis < 1 µm : ≥ 75 und ≤ 95 Vol.-% und
Poren mit Durchmesser im Bereich ≥ 1 bis ≤ 10 µm : ≥ 0 und ≤ 5 Vol.-%.

Das Porengesamtvolumen beträgt für erfindungsgemäß relevante Erststufenkatalysatoraktivmassen typisch 0,1 bis 1,00 ml/g, meist 0,10 bis 0,80 ml/g, bzw. 0,20 bis 0,40 ml/g.

Weiterhin ist das erfindungsgemäße Verfahren von besonderer Relevanz, wenn die Aktivmasse für die Katalysatoren der zweiten Reaktionsstufe solche umfassen, deren spezifische Oberfläche 0,1 bis 150 m²/g, bzw. 0,2 bis 50 m²/g, bzw. 1 bis 20 m²/g, oder 2 bis 10 m²/g beträgt. Zusätzlich ist das erfindungsgemäße Verfahren von besonderer Relevanz, wenn die Aktivmassen für die Katalysatoren der zweiten Reaktionsstufe solche umfassen, deren numerisch häufigster Porendurchmesser 0,1 bis 1 µm beträgt.

Es ist des weiteren von besonderer Relevanz, wenn bei den Aktivmassen für die Katalysatoren der zweiten Reaktionsstufe vorgenannter numerisch häufigster Porendurchmesser und eine der vorgenannten spezifischen Oberflächen kombiniert vorliegen.

Das Porengesamtvolumen beträgt für erfindungsgemäß relevante Zweitstufenkatalysatoren typisch 0,10 bis 0,90 ml/g, bzw. 0,20 bis 0,80 ml/g, oder 0,30 bis 0,70 ml/g.

Außerdem ist das erfindungsgemäße Verfahren von besonderer Bedeutung, wenn bei den Aktivmassen für die Katalysatoren der zweiten Reaktionsstufe die Porenverteilung so beschaffen ist, dass auf die Durchmesserbereiche 0 bis < 1,0 µm, 1,0 bis < 10 µm und 10 µm bis 100 µm jeweils wenigstens 5 Vol.-%, bevorzugt wenigstens 10 Vol.-% des vorgenannten Porengesamtvolumens entfallen:

Vorteilhaft ist die erfindungsgemäße Verfahrensweise auch dann, wenn die Porendurchmesser-Verteilungen gemäß der EP-A 293 859 bei den Zweitstufenkatalysatoraktivmassen vorliegen. Jede einzelne der vorstehend gemachten Aussagen zu spezifischer Oberfläche, Porendurchmesser, Porengesamtvolumen und Pörendurchmesser-Verteilung gilt insbesondere im Bezug auf jede einzelne in dieser Schrift als relevant erwähnte Multimetalloxidmasse für Katalysatoren der ersten Oxidationsstufe und der zweiten Oxidationsstufe.

Prinzipiell kann beim erfindungsgemäßen Verfahren innerhalb der ersten Reaktionsstufe die volumenspezifische Aktivität des wenigstens einen ersten Katalysatorbetts (insbesondere Katalysatorfestbetts) in Strömungsrichtung das Reaktionsgasgemischs 1 über die Länge des Strömungsweges entweder konstant sein, oder wenigstens einmal (kontinuierlich, oder abrupt oder stufenförmig) zunehmen. Dabei ist die wenigstens einmalige Zunahme unter den erfindungsgemäßen Gesichtspunkten (möglichst geringe Nebenproduktbildung) erfindungsgemäß bevorzugt. In allen vorgenannten Fällen ist es ferner von Vorteil, wenn sich die Aktivmassenzusammensetzung über die Länge des Strömungsweges innerhalb der ersten Reaktionsstufe nicht ändert.

Das für die erste Reaktionsstufe Vorgenannte gilt ebenso für die zweite Reaktionsstufe des erfindungsgemäßen Verfahrens.

Ist das Katalysatorbett für die erste Reaktionsstufe ein Katalysatorfestbett, so können zur Bereitung dieser Festbettkatalysatorschüttung 1 beim erfindungsgemäßen Verfahren nur Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden, Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit in Betracht.

Die Geometrie solcher inerter Verdünnungsformkörper kann im Prinzip beliebig sein. D.h., es können beispielsweise Kugeln, Polygone, Vollzylinder oder auch, wie die Aktivmasse aufweisenden Katalysatorformkörper, Ringe sein. Erfindungsgemäß bevorzugt wird man als inerte Verdünnungsformkörper solche wählen, deren Geometrie derjenigen der mit ihnen zu verdünnenden Katalysatorformkörper entspricht (vorgenannte Aussagen gelten so auch für zur Bereitung einer Festbettkatalysatorschüttung 2 (Katalysatorfestbett für die zweite Reaktionsstufe) verwendbare weitgehend homogene Gemische aus Multimetalloxidaktivmasse aufweisenden Katalysatorformkörpern und Verdünnungsformkörpern).

Vorteilhaft ist es, wenn sich die chemische Zusammensetzung der verwendeten Aktivmasse über die Festbettkatalysatorschüttung 1 nicht verändert. D.h., die für einen einzelnen Katalysatorformkörper verwendete Aktivmasse kann zwar ein Gemisch aus verschiedenen Multimetalloxiden sein, für alle Katalysatorformkörper der Festbettkatalysatorschüttung 1 ist dann jedoch das gleiche Gemisch zu verwenden.

Die volumenspezifische (d.h., die auf die Einheit des Volumens normierte) Aktivität kann in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen der Schüttung enthaltene Aktivmasse bzw. Katalysatoraktivität.

Eine in Strömungsrichtung des Reaktionsgasgemisches über die Festbettkatalysatorschüttung 1 wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Lässt man den Anteil an Verdünnungsformkörpern konstant oder werden überhaupt keine Verdünnungsformkörper in der Festbettkatalysatorschüttung 1 mitverwendet, resultiert eine konstante volumenspezifische Aktivität in Strömungsrichtung des Reaktionsgasgemisches über die Festbettkatalysatorschüttung 1. Eine Zunahme der volumenspezifischen Aktivität ist aber auch z.B. dadurch möglich, dass man bei gleichbleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Im Normalfall wird beim erfindungsgemäßen Verfahren weder innerhalb der Festbettkatalysatorschüttung 1 noch innerhalb der Festbettkatalysatorschüttung 2 in Strömungsrichtung des Reaktionsgasgemisches die volumenspezifische Aktivität einmal abnehmen.

Vorab und/oder im Anschluss an die Festbettkatalysatorschüttung 1 können sich ausschließlich aus Inertmaterial (z.B. nur Verdünnungsformkörpern) bestehende Schüttungen befinden (sie werden in dieser Schrift begrifflich nicht der Festbettkatalysatorschüttung 1 zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung 1 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Erfindungsgemäß bevorzugt ist beim erfindungsgemäßen Verfahren die Festbettkatalysatorschüttung 1 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z.B. auf einer Länge von 0,70 bis 1,50 m, bevorzugt 0,90 bis 1,20 m), jeweils der Gesamtlänge der Festbettkatalysatorschüttung 1 entweder nur Katalysatorformkörper, oder ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung 1 befindet sich dann erfindungsgemäß vorteilhaft bis zum Ende der Länge der Festbettkatalysatorschüttung 1 (d.h., z.B. auf einer Länge von 2,00 bis 3,00 m; bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind.

Das Vorgenannte trifft insbesondere dann zu, wenn in der Festbettkatalysatorschüttung 1 als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

In entsprechender Weise wie die volumenspezifische Aktivität der Festbettkatalysatorschüttung 1 variiert werden kann, kann auch die volumenspezifische Aktivität der Festbettkatalysatorschüttung 2 variiert werden. Dabei kann sich vorab und/oder im Anschluss an die eigentliche Festbettkatalysatorschüttung 2 wiederum eine entsprechende Inertschüttung befinden.

Erfindungsgemäß bevorzugt ist beim erfindungsgemäßen Verfahren die Festbettkatalysatorschüttung 2 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Festbettkatalysatorschüttung 2, entweder nur Katalysatorformkörper oder ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.-% und besonders bevorzugt 25 bis 35 Gew.-% beträgt. Im Anschluss an diese erste Zone der Festbettkatalysatorschüttung 2 befindet sich dann erfindungsgemäß vorteilhaft bis zum Ende der Länge der Festbettkatalysatorschüttung 2 (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn in der Festbettkatalysatorschüttung 2 als Katalysatorformkörper Schalenkatalysatorringe eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Anwendungstechnisch zweckmäßig kann die Durchführung der ersten Reaktionsstufe des erfindungsgemäßen Verfahrens z.B. in einem mit der Festbettkatalysatorschüttung 1 (sowie gegebenenfalls dieser vorangehenden und/oder nachfolgenden Inertschüttungen) beschickten Rohrbündelreaktor, wie er z.B. in der EP-B 700714 beschrieben ist, erfolgen.

D.h., in einfachster Weise befindet sich in den einzelnen Metallrohren eines Rohrbündelreaktors jeweils die vorgenannte Beschickung und um die Metallrohre wird ein Temperaturmedium (Einabschnittfahrweise), in der Regel eine Salzschmelze, geführt. Salzschmelze und Reaktionsgasgemisch können dabei im einfachen Gleich- oder Gegenstrom geführt werden. Die Salzschmelze (das Temperiermedium) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemisches besteht. Die Strömungsgeschwindigkeit des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittsstelle in den Reaktor bis zur Austrittsstelle aus dem Reaktor ≥ 0 bis 10°C, häufig ≥ 2 bis 8°C, oft ≥ 3 bis 6°C beträgt. Die Eintrittstemperatur des Wärmeaustauschmittels in den Rohrbündelreaktor beträgt in der Regel 300 bis 360°C, häufig 300 bis 340°C.

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt (vor allem bei Anwendung der in dieser Schrift genannten Katalysatorringgeometrien) in der Regel 20 bis 30 mm, häufig 21 bis 26 mm. Ihre Länge liegt typisch bei 2 bis 4 m, häufig bei 2,5 bis 3,5 m. Davon werden erfindungsgemäß normalerweise wenigstens 60 %, häufig wenigstens 75 % von der Festbettkatalysatorschüttung 1 belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000, oder bis 40000. Rohrbündelreaktoren mit mehr als 50000 Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290). Einen für das erfindungsgemäße Verfahren geeigneten Rohrbündelreaktor offenbaren auch die DE-A 10131126, die DE-A 10137768, die DE-A 10135498 und die DE-A 10232967.

Zweckmäßigerweise wird das Reaktionsgasausgangsgemisch 1 der Festbettkatalysatorschüttung 1 auf die Reaktionstemperatur vorerwärmt zugeführt. Diesem Zweck kann z.B. eine einer Festbettkatalysatorschüttung vorausgehende Schüttung mit Inertmaterial dienen.

Selbstredend kann die erste Reaktionsstufe des erfindungsgemäßen Verfahrens auch in einem Zwei(oder mehr)abschnittrohrbündelreaktor durchgeführt werden, wie er z.B. in den DE-A's 19910508, 19948523, 19910506 und 19948241 beschrieben ist. Eine bevorzugte Variante eines erfindungsgemäß einsetzbaren Zweiabschnittrohrbündelreaktors offenbart die DE-C 2830765. Aber auch die in der DE-C 2513405, der US-A 3147084, der DE-A 2201528, der EP-A 383224 und der DE-A 2903218 offenbarten Zweiabschnittrohrbündelreaktoren sind für eine Durchführung der ersten Reaktionsstufe des erfindungsgemäßen Verfahrens geeignet.

D.h., in einfachster Weise befindet sich die erfindungsgemäß zu verwendende Festbettkatalysatorschüttung 1 (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß einen Reaktionsabschnitt. D.h., in einfachster Weise umströmt z.B. ein Salzbad A denjenigen Abschnitt der Rohre (den Reaktionsabschnitt A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (den Reaktionsabschnitt B), in welchem sich die oxidative Anschlussumsetzung des Propens (beim einfachen Durchgang) bis zum Erreichen eines Umsatzwertes von wenigstens 90 mol-% vollzieht (bei Bedarf können sich an die erfindungsgemäß anzuwendenden Reaktionsabschnitte A, B weitere Reaktionsabschnitte anschließen, die auf individuellen Temperaturen gehalten werden).

Anwendungstechnisch zweckmäßig umfasst die erste Reaktionsstufe des erfindungsgemäßen Verfahrens keine weiteren Reaktionsabschnitte. D.h., das Salzbad B umströmt zweckmäßig den Abschnitt der Rohre, in welchem sich die oxidative Anschlussumsetzung des Propens (beim einfachen Durchgang) bis zu einem Umsatzwert ≥ 90 mol-%, oder ≥ 92 mol-%, oder ≥ 94 mol-%, oder ≥ 96 mol-% oder mehr vollzieht.

Üblicherweise liegt der Beginn des Reaktionsabschnitts B hinter dem Heißpunktmaximum des Reaktionsabschnitts A. Das Heißpunktmaximum des Reaktionsabschnitts B liegt normalerweise unterhalb der Heißpunktmaximaltemperatur des Reaktionsabschnitts A.

Die beiden Salzbäder A,B können erfindungsgemäß relativ zur Strömungsrichtung des durch die Reaktionsrohre strömenden Reaktionsgasgemischs im Gleichstrom oder im Gegenstrom durch den die Reaktionsrohre umgebenden Raum geführt werden. Selbstverständlich kann erfindungsgemäß auch im Reaktionsabschnitt A eine Gleichströmung und im Reaktionsabschnitt B eine Gegenströmung (oder umgekehrt) angewandt werden.

Selbstverständlich kann man in allen vorgenannten Fallkonstellationen innerhalb des jeweiligen Reaktionsabschnitts der, relativ zu den Reaktionsrohren, erfolgenden Parallelströmung der Salzschmelze noch eine Querströmung überlagern, so dass der einzelne Reaktionsabschnitt einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird auch bei der Zweiabschnittfahrweise das Reaktionsgasausgangsgemisch 1 der Festbettkatalysatorschüttung 1 auf die Reaktionstemperatur vorerwärmt zugeführt.

Üblicherweise sind auch in den Zweiabschnittrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jedem Temperaturabschnitt belegt die Festbettkatalysatorschüttung 1 wenigstens 60 % bzw. wenigstens 75 %, oder wenigstens 90 % der Länge des Abschnitts. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000 oder bis 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriümnitrat, oder von niedrig schmelzenden Metallen wie Natrium Quecksilber sowie Legierungen verschiedener Metalle.

In der Regel wird bei allen vorstehend erwähnten Konstellationen der Stromführung in den Zweiabschnittrohrbündelreaktoren die Fließgeschwindigkeit innerhalb der beiden erforderlichen Wärmeaustauschmittelkreisläufe so gewählt, dass die Temperatur des Wärmeaustauschmittels von der Eintrittsstelle in den Reaktionsabschnitt bis zur Austrittstelle aus dem Reaktionsabschnitt (bedingt durch die Exothermie der Reaktion) um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in den Reaktionsabschnitt A beträgt erfindungsgemäß normalerweise 300 bis 340°C. Die Eintrittstemperatur des Wärmeaustauschmittels in den Reaktionsabschnitt B beträgt erfindungsgemäß normalerweise einerseits 305 bis 380°C und liegt andererseits gleichzeitig wenigstens ≥ 0°C, oder wenigstens 5°C oberhalb der Eintrittstemperatur des in den Reaktionsabschnitt A eintretenden Wärmeaustauschmittels. Gegebenenfalls kann dieser Temperaturunterschied auch ≤ 0°C betragen.

Bei hohen Propenlasten liegt die Eintrittstemperatur des Wärmeaustauschmittels in den Reaktionsabschnitt B zweckmäßig wenigstens 10°C oberhalb der Eintrittstemperatur des in den Reaktionsabschnitt A eintretenden Wärmeaustauschmittels. Die Differenz zwischen den Eintrittstemperaturen in den Reaktionsabschnitt A bzw. B kann erfindungsgemäß somit bis zu 20°C, bis zu 25°C, bis zu 30°C, bis zu 40°C, bis zu 45°C oder bis zu 50°C betragen. Normalerweise wird die vorgenannte Temperaturdifferenz aber nicht mehr als 50°C betragen. Je höher die Propenbelastung der Festbettkatalysatorschüttung 1 beim erfindungsgemäßen Verfahren gewählt wird, um so größer sollte die Differenz zwischen der Eintrittstemperatur des Wärmeaustauschmittels in den Reaktionsabschnitt A und der Eintrittstemperatur des Wärmeaustauschmittels in den Reaktionsabschnitt B sein.

Mit Vorteil beträgt die Eintrittstemperatur des Wärmeaustauschmittels in den Reaktionsabschnitt B erfindungsgemäß 330 bis 370°C und besonders vorteilhaft 340 bis 370°C.

Selbstverständlich können beim erfindungsgemäßen Verfahren die beiden Reaktionsabschnitte A, B auch in räumlich voneinander getrennten Rohrbündelreaktoren realisiert sein. Bei Bedarf kann zwischen den beiden Reaktionsabschnitten A, B auch ein Wärmetauscher angebracht werden.

Es sei an dieser Stelle auch noch einmal darauf hingewiesen, dass für eine Durchführung der Reaktionsstufe 1 des erfindungsgemäßen Verfahrens insbesondere auch der in der DE-AS 2201528 beschriebene Zweiabschnittrohrbündelreaktortyp verwendet werden kann, der die Möglichkeit beinhaltet, vom heißeren Wärmeaustauschmittel des Reaktionsabschnitts B eine Teilmenge an den Reaktionsabschnitt A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb eines individuellen Reaktionsabschnitts wie in der EP-A 382098 beschrieben gestaltet werden.

Erfindungsgemäß hat es sich als zweckmäßig erwiesen, das die erste Reaktionsstufe verlassende Produktgasgemisch vor dem Eintritt in die zweite Reaktionsstufe abzukühlen, um so eine Nachvollverbrennung von Teilen des in der ersten Reaktionsstufe gebildeten Acroleins zu unterdrücken. Üblicherweise wird dazu zwischen die beiden Reaktionsstufen ein Nachkühler geschaltet. Dies kann im einfachsten Fall ein indirekter Rohrbündelwärmeüberträger sein. Das Produktgasgemisch wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z.B. Spiralen aus Edelstahl, Ringe aus Steatit, Kugeln aus Steatit etc.) gefüllt. Selbige verbessern den Wärmeaustausch und fangen gegebenenfalls aus der Festbettkatalysatorschüttung der ersten Reaktionsstufe sublimierendes Molybdäntrioxid vor einem Eintritt desselben in die zweite Reaktionsstufe ab. Es ist von Vorteil, wenn der Nachkühler aus mit Zinksilicatfarbe beschichtetem rostfreiem Stahl gefertigt ist.

In der Regel wird der auf den einfachen Durchgang bezogene Propenumsatz beim erfindungsgemäßen Verfahren in der ersten Reaktionsstufe ≥ 92 mol-% oder ≥ 94 mol-%, oder > 96 mol-%, oder mehr betragen. Die in der ersten Reaktionsstufe bei einfachem Durchgang resultierende Selektivität S^{AA} der Acroleinbildung sowie der Acrylsäurenebenproduktbildung wird dabei erfindungsgemäß zusammen regelmäßig ≥ 85 mol-% bzw. ≥ 90 mol-%, vielfach ≥ 92 mol-% oder ≥ 94 mol-%, häufig ≥ 95 mol-% oder ≥ 96 mol-% bzw. ≥ 97 mol-% betragen.

Das erfindungsgemäße Verfahren eignet sich für Propenbelastungen der Festbettkatalysatorschüttung 1 von ≥ 80 NI/I·h, oder von ≥ 100 NI/I·h, oder von ≥ 120 NI/I·h, oder von ≥ 140 NI/I·h, oder von ≥ 165 NI/I·h, oder von ≥ 170 NI/I·h bzw. ≥ 175 NI/I·h oder ≥ 180 NI/I·h, aber auch für Propenbelastungen der Festbettkatalysatorschüttung 1 von ≥ 185 NI/I·h, oder ≥ 190 NI/I·h bzw. ≥ 200 NI/I·h oder ≥ 210 NI/I·H sowie für Belastungswerte von ≥ 220 NI/I·h oder ≥ 230 NI/I·h bzw. ≥ 240 NI/I·h oder ≥ 250 NI/I·h.

Mit zunehmender Propenbelastung ist die beschriebene Zweiabschnittfahrweise gegenüber der beschriebenen Einabschnittsfahrweise in der ersten Reaktionsstufe bevorzugt.

Normalerweise wird beim erfindungsgemäßen Verfahren die Propenbelastung der ersten Festbettkatalysatorschüttung 600 NI/I·h nicht überschreiten. In typischer Weise liegen die Propenbelastungen der Festbettkatalysatorschüttung 1 beim erfindungsgemä-βen Verfahren bei Werten ≤ 300 NI/I·h, häufig bei Werten ≤ 250 NI/I·h.

Der Arbeitsdruck kann beim erfindungsgemäßen Verfahren in der ersten Reaktionsstufe sowohl unterhalb von Normaldruck (z.B. bis zu 0,5 bar, das Reaktionsgasgemisch wird durchgesaugt) als auch oberhalb von Normaldruck liegen. Typischerweise wird der Arbeitsdruck in der ersten Reaktionsstufe bei Werten von 1 bis 5 bar, häufig 1,5 bis 3,5 bar liegen. Normalerweise wird der Reaktionsdruck in der ersten Reaktionsstufe 100 bar nicht überschreiten.

Als Quelle für den in der ersten Reaktionsstufe erforderlichen molekularen Sauerstoff kommt sowohl Luft, als auch an molekularem Stickstoff entreicherte Luft in Betracht.

Anwendungstechnisch zweckmäßig wird das Produktgasgemisch der ersten Reaktionsstufe im bereits erwähnten Nachkühler auf eine Temperatur von 210 bis 290°C, häufig 230 bis 280°C oder 250 bis 270°C abgekühlt. Dabei kann die Abkühlung des Produktgasgemisches der ersten Reaktionsstufe durchaus auf Temperaturen erfolgen, die unterhalb der Temperatur der zweiten Reaktionsstufe liegen. Die beschriebene Nachkühlung ist jedoch keineswegs zwingend und kann insbesondere dann in aller Regel entfallen, wenn der Weg des Produktgasgemisches von der ersten Reaktionsstufe in die zweite Reaktionsstufe kurz gehalten wird. Üblicherweise wird das erfindungsgemäße Verfahren ferner so verwirklicht, dass man den Sauerstoffbedarf in der zweiten Reaktionsstufe nicht bereits durch einen entsprechend hohen Sauerstoffgehalt des Reaktionsgasausgangsgemisches 1 deckt, sondern den benötigten Sauerstoff im Bereich zwischen erster und zweiter Reaktionsstufe zugibt. Dies kann vor, während, nach und/oder zur Nachkühlung erfolgen. Als Quelle für den in der zweiten Reaktionsstufe erforderlichen molekularen Sauerstoff kommen sowohl reiner Sauerstoff als auch Gemische aus Sauerstoff und Inertgas, z.B. Luft oder an molekularem Stickstoff entreicherte Luft (z.B. ≥ 90 Vol-% O₂, ≤ 10 Vol-% N₂) in Betracht. Die Zugabe der Sauerstoffquelle erfolgt regelmäßig in auf den Reaktionsdruck komprimierter Form. Selbstredend kann beim erfindungsgemäßen Verfahren der Sauerstoffbedarf in der zweiten Reaktionsstufe bereits durch einen entsprechend hohen Sauerstoffbedarf in der ersten Reaktionsstufe gedeckt werden.

Der Arbeitsdruck kann beim erfindungsgemäßen Verfahren in der zweiten Reaktionsstufe wie in der Reaktionsstufe 1 sowohl unterhalb von Normaldruck (z.B. bis zu 0,5 bar) als auch oberhalb von Normaldruck liegen. Typischerweise wird der Arbeitsdruck in der zweiten Reaktionsstufe erfindungsgemäß bei Werten von 1 bis 5 bar, häufig 1 bis 3 bar liegen. Normalerweise wird der Reaktionsdruck in der zweiten Reaktionsstufe 100 bar nicht überschreiten.

Ebenso wie die erste Reaktionsstufe kann die zweite Reaktionsstufe des erfindungsgemäßen Verfahrens in einfacher Weise in einem mit der Festbettkatalysatorschüttung 2 beschickten Rohrbündelreaktor, wie er z.B. in der EP-A 700893 beschrieben ist, durchgeführt werden. Der Festbettkatalysatorschüttung 2 vorausgehende und/oder nachfolgende Inertschüttungen können die Beschickung ergänzen.

D.h., in einfachster Weise befindet sich der erfindungsgemäß zu verwendende Festbettkatalysator 2 sowie die gegebenenfalls mitverwendeten Inertschüttungen in den Metallrohren eines Rohrbündelreaktors und um die Metallrohre wird ein Temperiermedium (Einabschnittfahrweise), in der Regel eine Salzschmelze geführt. Salzschmelze und Reaktionsgasgemisch können dabei im einfachen Gleich- oder Gegenstrom geführt werden. Das Temperiermedium kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemisches besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor ≥ 0 bis 10°C, häufig ≥ 2 bis 8°C, oft ≥ 3 bis 6°C beträgt. Die Eintrittstemperatur des Wärmeaustauschmittels in den Rohrbündelreaktor beträgt in der Regel 230 bis 300°C, häufig 245 bis 285°C bzw. 255 bis 275°C. Als Wärmeaustauschmittel eignen sich dabei die gleichen fluiden Temperiermedien, wie sie bereits für die erste Reaktionsstufe beschrieben worden sind.

Zweckmäßigerweise wird das Reaktionsgasausgangsgemisch 2 der Festbettkatalysatorschüttung 2 auf die Reaktionstemperatur vorerwärmt zugeführt. Für die Dimensionierung der Kontaktrohre, das Kontaktrohrmaterial, die Kontäktrohranzahl und ihre Beschickung mit Festbettkatalysatorschüttung 2/Inertschüttung gilt das für den Rohrbündelreaktor der ersten Reaktionsstufe Gesagte.

In der Regel wird eine Einabschnittfahrweise der ersten Reaktionsstufe mit einer Einabschnittfahrweise der zweiten Reaktionsstufe kombiniert, wobei die relative Stromführung von Reaktionsgasgemisch und Temperiermedium in beiden Stufen identisch gewählt wird.

Selbstverständlich kann aber auch die zweite Reaktionsstufe des erfindungsgemäßen Verfahrens in entsprechender Weise wie die erste Reaktionsstufe als zwei räumlich aufeinander folgende Reaktionsabschnitte C, D realisiert werden, wobei die Temperatur des Reaktionsabschnitts C (gemeint ist damit stets die Temperatur des eintretenden Salzbades bzw. allgemein Wärmeträgers) zweckmäßig 230 bis 270°C und die Temperatur des Reaktionsabschnitts D 250 bis 300°C beträgt und gleichzeitig wenigstens ≥ 0°C, oder wenigstens ≥ 5°C oberhalb der Temperatur der Reaktionszone C liegt. Gegebenenfalls kann dieser Temperaturunterschied aber auch ≤ 0°C betragen.

Bevorzugt erstreckt sich der Reaktionsabschnitt C bis zu einem Acroleinumsatz von 65 bis 80 mol-%. Außerdem liegt die Temperatur des Reaktionsabschnitts C mit Vorteil bei 245 bis 260°C. Die Temperatur des Reaktionsabschnitts D liegt bei hohen Acroleinlasten vorzugsweise 5 bis 10°C oberhalb der Temperatur des Reaktionsabschnitts C und beträgt vorteilhaft 260 bis 285°C. Auch für die Zweiabschnittfahrweise der zweiten Reaktionsstufe gilt bezüglich des Reaktors für die Dimensionierung der Kontaktrohre, das Kontaktrohrmaterial, die Kontaktrohranzahl und ihre Beschickung mit Festbettkatalysator 2/Inertschüttung das für den Zweiabschnittrohrbündelreaktor der ersten Reaktionsstufe Gesagte.

Je höher die Acroleinbelastung der Festbettkatalysatorschüttung 2 beim erfindungsgemäßen Verfahren gewählt wird, um so mehr ist die Zweiabschnittfahrweise gegenüber der Einabschnittfahrweise bevorzugt und umso größer sollte die Differenz zwischen der Temperatur des Reaktionsabschnitts C und der Temperatur des Reaktionsabschnitts D gewählt werden. Normalerweise wird die vorgenannte Temperaturdifferenz aber nicht mehr als 40°C betragen. D.h., die Differenz zwischen der Temperatur des Reaktionsabschnitts C und der Temperatur des Reaktionsabschnitts D kann erfindungsgemäß bis zu 15°C, bis zu 25°C, bis zu 30°C, bis zu 35°C oder bis zu 40°C betragen.

Generell kann beim erfindungsgemäßen Verfahren der auf den einfachen Durchgang der zweiten Reaktionsstufe bezogene Acroleinumsatz ≥ 90 mol-%, oder ≥ 92 mol-%, oder ≥ 94 mol-%, oder ≥ 96 mol-%, oder ≥ 98 mol-% und häufig sogar ≥ 99 mol-% betragen. Die Selektivität der Acrylsäurebildung, bezogen auf umgesetztes Acrolein, kann dabei regelmäßig ≥ 92 mol-%, bzw. ≥ 94 mol-%, häufig ≥ 95 mol-% oder ≥ 96 mol-% bzw. ≥ 97 mol-% betragen.

Das erfindungsgemäße Verfahren eignet sich für Acroleinbelastungen der Festbettkatalysatorschüttung 2 von ≥ 80 Nl/l·h, oder von ≥ 100 Nl/l·h, oder von ≥ 120 Nl/l·h, oder von ≥ 140 Nl/l·h bzw. ≥ 150 Nl/l·h, oder von ≥ 160 Nl/l·h bzw. ≥ 170 Nl/l·h, oder ≥ 175 Nl/l·h bzw. ≥ 180 Nl/l·h, aber auch bei Acroleinbelastungen der Festbettkatalysatorschüttung 2 von ≥ 185 Nl/l·h, oder von ≥ 190 Nl/l·h bzw. ≥ 200 Nl/l·h, oder ≥ 210 Nl/l·h sowie bei Belastungswerten ≥ 220 Nl/l·h, oder ≥ 230 Nl/l·h bzw. 240 Nl/l·h, oder ≥ 250 Nl/l·h.

Erfindungsgemäß bevorzugt, wird zwischen der ersten und der zweiten Reaktionsstufe kein nur aus Inertgas bestehendes Sekundärgas zudosiert.

Normalerweise wird beim erfindungsgemäßen Verfahren die Acroleinbelastung der zweiten Festbettkatalysatorschüttung den Wert von 600 Nl/l·h nicht überschreiten. In typischer Weise liegen die Acroleinbelastungen der Festbettkatalysatorschüttung 2 beim erfindungsgemäßen Verfahren ohne nennenswerten Verlust von Umsatz und Selektivität bei Werten ≤ 300 Nl/l·h, häufig bei Werten ≤ 250 Nl/l·h.

In der Regel wird beim erfindungsgemäßen Verfahren die Acroleinbelastung der Festbettkatalysatorschüttung 2 etwa 10 Nl/l·h, häufig etwa 20 bzw. 25 Nl/l·h unterhalb der Propenbelastung der Festbettkatalysatorschüttung 1 liegen. Dies ist primär darauf zurückzuführen, dass in der ersten Reaktionsstufe sowohl Umsatz als auch Selektivität zu Acrolein in der Regel nicht 100 % erreichen. Ferner wird der Sauerstoffbedarf der zweiten Reaktionsstufe üblicherweise durch Luft als Sekundärgas gedeckt. Mit zunehmender Acroleinbelastung ist die beschriebene Zweiabschnittfahrweise gegenüber der ausgeführten Einabschnittfahrweise in der zweiten Reaktionsstufe bevorzugt.

Beachtenswerterweise kann beim erfindungsgemäßen Verfahren die über beide Reaktionsstufen bilanzierte Selektivität der Acrylsäurebildung, bezogen auf umgesetztes Propen, selbst bei höchsten Propen- und Acroleinbelastungen in der Regel bei Werten ≥ 83 mol-%, häufig bei ≥ 85 mol-% oder ≥ 88 mol-%, oft bei ≥ 90 mol-% oder ≥ 93 mol-% oder mehr liegen.

In anwendungstechnisch zweckmäßiger Weise erfolgt die Durchführung der zweiten Reaktionsstufe des erfindungsgemäßen Verfahrens in einem Zweiabschnittrohrbündelreaktor. Eine bevorzugte Variante eines für die zweite Reaktionsstufe erfindungsgemäß einsetzbaren Zweiabschnittrohrbündelreaktors offenbart die DE-C 2830765. Aber auch die in der DE-C 2513405, der US-A 3147084, der DE-A 2201528, der EP-A 383224 und der DE-A 2903582 offenbarten Zweiabschnittrohrbündelreaktoren sind für eine Durchführung der zweiten Reaktionsstufe des erfindungsgemäßen Verfahrens geeignet.

D.h., in einfacher Weise befindet sich die erfindungsgemäß zu verwendende Festbettkatalysatorschüttung 2 (gegebenenfalls einschließlich der Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß einen Reaktionsabschnitt.

D.h., in einfacher Weise umströmt z.B. ein Salzbad C diejenigen Abschnitte der Rohre (den Reaktionsabschnitt C), in welchem sich die oxidative Umsetzung des Acroleins (beim einfachen Durchgang) bis zum Erreichen eines Umsatzwertes im Bereich von 55 bis 85 mol.-% vollzieht und ein Salzbad D umströmt den Abschnitt der Rohre (den Reaktionsabschnitt D), in welchem sich die oxidative Anschlussumsetzung des Acroleins (beim einfachen Durchgang) bis zum Erreichen eines Umsatzwertes von wenigstens 90 mol.-% vollzieht (bei Bedarf können sich an die erfindungsgemäß anzuwendenden Reaktionszonen C,D weitere Reaktionszonen anschließen, die auf individuellen Temperaturen gehalten werden).

Anwendungstechnisch zweckmäßig umfasst die Reaktionsstufe 2 des erfindungsgemäßen Verfahrens keine weiteren Reaktionsabschnitte. D.h., das Salzbad D umströmt zweckmäßig den Abschnitt der Rohre, in welchem sich die oxidative Anschlussumsetzung des Acroleins (beim einfachen Durchgang) bis zu einem Umsatzwert von ≥ 92 mol.-%, oder ≥ 94 mol.-%, oder ≥ 96 mol.-%, oder ≥ 98 mol.-%, und häufig sogar ≥ 99 mol.-% oder mehr vollzieht.

Üblicherweise liegt der Beginn des Reaktionsabschnitts D hinter dem Heißpunktmaximum des Reaktionsabschnitts C. Die Temperatur des Heißpunktmaximums des Reaktionsabschnitts D liegt normalerweise unterhalb der Heißpunktmaximaltemperatur des Reaktionsabschnitts C.

Die beiden Salzbäder C, D können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch im Reaktionsabschnitt C eine Gleichströmung und im Reaktionsabschnitt D eine Gegenströmung (oder umgekehrt) angewandt werden.

Selbstredend kann man in allen vorgenannten Fallkonstellationen innerhalb des jeweiligen Reaktionsabschnitts der, relativ zu den Reaktionsrohren, erfolgenden Parallelströmung der Salzschmelze noch eine Querströmung überlagern, so dass der einzelne Reaktionsabschnitt einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Üblicherweise sind in den vorgenannten Zweizonen-Rohrbündelreaktoren (ebenso wie in den Rohrbündelreaktoren der Einzonenfahrweise) die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Ihre Länge beträgt zweckmäßig 3 bis 4, bevorzugt 3,5 m. In jeder Temperaturzone belegt die Festbettkatalysatorschüttung 2 wenigstens 60 %, bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000 oder 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweiabschnittrohrbündelreaktoren die Fließgeschwindigkeit innerhalb der beiden erforderlichen Wärmeaustauschmittelkreisläufe so gewählt, dass die Temperatur des Wärmeaustauschmittels von der Eintrittstelle in die Reaktionszone bis zur Austrittsstelle aus der Reaktionszone um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in den Reaktionsabschnitt C beträgt bei einer erfindungsgemäßen Zweiabschnittfahrweise in der zweiten Reaktionsstufe normalerweise 230 bis 270°C. Die Eintrittstemperatur des Wärmeaustauschmittels in den Reaktionsabschnitt D beträgt dabei erfindungsgemäß normalerweise einerseits 250 bis 300°C und liegt andererseits gleichzeitig wenigstens ≥ 0°C, oder wenigstens ≥ 5°C oberhalb der Eintrittstemperatur des in den Reaktionsabschnitt C eintretenden Wärmeaustauschmittels.

Bei hohen Acroleinlasten liegt die Eintrittstemperatur des Wärmeaustauschmittels in den Reaktionsabschnitt D bevorzugt 5 bis 10°C oberhalb der Eintrittstemperatur des in den Reaktionsabschnitt C eintretenden Wärmeaustauschmittels. Die Differenz zwischen den Eintrittstemperaturen in den Reaktionsabschnitt C bzw. D kann erfindungsgemäß aber auch bis zu 15°C, bis zu 25°C, bis zu 30°C, bis zu 35°C oder bis zu 40°C betragen. Normalerweise wird die vorgenannte Temperatur aber nicht mehr als 50°C betragen. Je höher die Acroleinbelastung der Katalysatorschüttung 2 beim erfindungsgemäßen Verfahren gewählt wird, um so größer sollte die Differenz zwischen der Eintrittstemperatur des Wärmeaustauschmittels in den Reaktionsabschnitt C und der Eintrittstemperatur des Wärmeaustauschmittels in den Reaktionsabschnitt D sein. Bevorzugt liegt die Eintrittstemperatur des Wärmeaustauschmittels in den Reaktionsabschnitt C bei 245 bis 260°C und die Eintrittstemperatur in den Reaktionsabschnitt D bei 260 bis 285°C.

Selbstverständlich können beim erfindungsgemäßen Verfahren die beiden Reaktionsabschnitte C, D auch in räumlich voneinander getrennten Rohrbündelreaktoren realisiert sein. Bei Bedarf kann zwischen den beiden Reaktionsabschnitten C, D auch ein Wärmetauscher angebracht werden.

Es sei an dieser Stelle auch noch einmal darauf hingewiesen, dass für eine Durchführung der zweiten Reaktionsstufe des erfindungsgemäßen Verfahrens insbesondere auch der in der DE-AS 2201528 beschriebene Zweiabschnittrohrbündelreaktortyp verwendet werden kann, der die Möglichkeit beinhaltet, vom heißeren Wärmeaustauschmittel des Reaktionsabschnitts D eine Teilmenge an den Reaktionsabschnitt C abzuführen, um gegebenenfalls ein Anwärmen eines zu kalten Reaktionsgasausgangsgemisches 2 oder eines kalten Kreisgases zu bewirken. Ferner kann die Rohrbündelcharakteristik innerhalb eines individuellen Reaktionsabschnitts wie in der EP-A 382 098 beschrieben gestaltet werden.

Selbstredend können beim erfindungsgemäßen Verfahren zwei Einabschnittrohrbündelreaktoren für die beiden Reaktionsstufen auch zu einem auf andere Art und Weise zu betreibenden einzelnen Zweiabschnittreaktor verschmolzen werden, wie es z. B. in der DE-C 2830765, in der EP-A 911313 sowie in der EP-A 383 224 beschrieben ist. In diesem Fall wird die erste Reaktionsstufe im ersten Reaktionsabschnitt und die zweite Reaktionsstufe im zweiten Reaktionsabschnitt des Zweizonenrohrbündelreaktors verwirklicht.

In völliger Entsprechung können auch ein Einabschnittrohrbündelreaktor und ein Zweiabschnittrohrbündelreaktor oder zwei Zweiabschnittrohrbündelreaktoren zu jeweils einem einzigen Rohrbündelreaktor verschmolzen werden, der dann drei bzw. vier Temperaturabschnitte aufweist und z. B. in der WO 01/36364 beschrieben ist.

In diesem Fall kann z. B. die erste Reaktionsstufe im ersten Reaktionsabschnitt und die zweite Reaktionsstufe in den beiden nachfolgenden Reaktionsabschnitten des Dreiabschnittrohrbündelreaktors durchgeführt werden. Alternativ kann z. B. die erste Reaktionsstufe in den ersten beiden Reaktionsabschnitten und die zweite Reaktionsstufe in den beiden nachfolgenden Reaktionsabschnitten des Vierabschnittrohrbündelreaktors durchgeführt werden u.s.w.. Die Salzbadtemperatur der einzelnen Temperaturabschnitte kann dabei wie im Fall der voneinander räumlich getrennten Rohrbündelreaktoren beschrieben gestaltet werden. Normalerweise befindet sich in diesen Fällen zwischen der Festbettkatalysatorschüttung 1 und der Festbettkatalysatorschüttung 2 eine Inertschüttung. Allerdings kann auf eine solche Zwischeninertschüttung auch verzichtet werden. Die Länge der Reaktionsrohre entspricht in den Verschmelzungsfällen vielfach der Summe der Längen der nicht verschmolzenen Rohrbündelreaktoren. Selbstredend kann das erfindungsgemäße Verfahren auch analog zur in den Schriften EP-A 990636 und EP-A 1106598 beschriebenen Verfahrensweisen ausgeführt werden.

In der Regel enthält das Reaktionsgasausgangsgemisch 1 beim erfindungsgemäßen Verfahren 3 bis 25 Vol.-%, vielfach 5 bis 20 Vol.-%, und meist 6 bis 13 Vol.-%, Propylen.

Der Gehalt an molekularem Sauerstoff im Reaktionsgasausgangsgemisch 1 ist erfindungsgemäß so zu bemessen, dass das molare Verhältnis V₁ von im Reaktionsgasausgangsgemisch 1 enthaltenem O₂ zu im Reaktionsgasausgangsgemisch enthaltenem C₃H₆ ≥ 1 beträgt. Üblicherweise beträgt V₁ beim erfindungsgemäßen Verfahren ≥ 1 und ≤ 3, meist ≥ 1,3 und ≤ 2,5, oft ≥ 1,5 bis ≤ 2,3. Die Menge an molekularem Sauerstoff im Reaktionsgasausgangsgemisch 2 wird normalerweise so bemessen, dass das molare Verhältnis von im Reaktionsgasausgangsgemisch 2 enthaltenem O₂ zu im Reaktionsgasausgangsgemisch 2 enthaltenem Acrolein ≥ 0,5 bis ≤ 2, häufig ≥ 0,75 bis ≤ 1,5 beträgt. Günstig ist es, wenn das Produktgasgemisch 2 noch bis zu 5, bzw. bis zu 3 Vol.-% molekularen Sauerstoff enthält.

Besonders relevant ist die erfindungsgemäße Verfahrensweise dann, wenn das Reaktionsgasausgangsgemisch 1 Wasserdampf enthält, da dieser die Wandlung des Cyclopropan fördert.

Auch kann das Reaktionsgasausgangsgemisch 1 bis zu 25 Vol.-% (z. B. 0,01 oder 0,1, oder 0,5, oder 2 bis 25 Vol.-%) an CO₂ enthalten.

Insbesondere dann, wenn als Quelle für den molekularen Sauerstoff beim erfindungsgemäßen Verfahren Luft verwendet wird, wird das Reaktionsgasausgangsgemisch 1 als inertes Verdünnungsgas molekularen Stickstoff enthalten. Grundsätzlich kann das Reaktionsgasausgangsgemisch 1 beim erfindungsgemäßen Verfahren ≥ 1 Vol.-%, oder ≥ 5 Vol.-%, oder ≥ 10 Vol.-%, oder ≥ 20 Vol.-%, oder ≥ 30 Vol.-%, oder ≥ 40 Vol.-% an molekularem Stickstoff enthalten. In der Regel wird der Gehalt des Reaktionsgasausgangsgemisches 1 an molekularem Stickstoff jedoch bei Werten ≤ 80 mol-%, oder ≤ 70 mol-%, oder ≤ 60 mol-% liegen.

Das erfindungsgemäße Verfahren umfasst daher insbesondere solche Ausführungsformen, bei denen das Reaktionsgasausgangsgemisch 1 > 0 bis 35 Vol.-%, häufig 1 bis 25 Vol.-%, oder 5 bis 15 Vol.-%, bzw. bis 10 Vol.-% H₂O enthält.

Typische Reaktionsgasausgangsgemische 1 sind z. B. solche, die enthalten:

| | |
|---|---|
| 6 bis 11 Vol.-% | Propen, |
| 6 bis 12 Vol.-% | Wasser, |
| ≥ 0 bis 5 Vol.-% | von Propen, Wasser, Sauerstoff, Cyclopropan und Stickstoff verschiedene Bestandteile, |

soviel molekularen Sauerstoff, dass V₁ 1 bis 3 beträgt, > 0 bis zu 3 mol-%, bezogen auf enthaltenes Propen, Cyclopropan, und als Restmenge bis zu 100 Vol.-% Gesamtmenge molekularen Stickstoff.

Das Reaktionsgasausgangsgemisch 1 kann erfindungsgemäß aber auch bis zu 70 Vol.-%, bzw. bis zu 60 Vol.-%, oder bis zu 50 Vol.%, oder bis zu 40 Vol.-%, oder bis zu 30 Vol.-%, oder bis zu 20 Vol.-%, oder bis zu 10 Vol.-% Propan enthalten. Häufig liegt dieser Propangehalt bei ≥ 0,01, bzw. ≥ 0,03, bzw. ≥ 0,05, bzw. ≥ 0,1, bzw. ≥ 1 Vol.-%. In der Regel enthält das Reaktionsgasausgangsgemisch 1 ≤ 10 Vol.-%, vielfach ≤ 5 Vol.-% an Propan. Dieses Propan kann beim erfindungsgemäßen Verfahren bewusst als inertes Verdünnungsgas zugegeben werden. Wird es als Verunreinigungen enthaltendes Roh-Propan zugesetzt, kann dieses auch Cyclopropan enthalten.

D. h., erfindungsgemäße Reaktionsgasausgangsgemische 1 können auch enthalten:

| | |
|---|---|
| 6 bis 9 Vol.-% | Propylen, |
| 8 bis 18 Vol.-% | molekularer Sauerstoff, |
| 6 bis 30 Vol.-% | Propan und |
| 32 bis 72 Vol.-% | molekularer Stickstoff. |

Erfindungsgemäße Reaktionsgasausgangsgemische 2 können enthalten:

| | |
|---|---|
| 4,5 bis 8 Vol.-% | Acrolein, |
| 2,25 bis 9 Vol.-% | molekularer Sauerstoff, |
| 6 bis 30 Vol.-% | Propan, |
| 32 bis 72 Vol.-% | molekularer Stickstoff, |
| 5 bis 30 Vol.-% | Wasserdampf. |

Erfindungsgemäße Reaktionsgasausgangsgemische 1 können aber auch bis zu 20 Vol.-% H₂ enthalten.

D.h., Reaktionsgasgemische 1 des erfindungsgemäßen Verfahrens können auch enthalten:

| | |
|---|---|
| 4 bis 25 Vol.-% | Propylen, |
| 6 bis 70 Vol.-% | Propan, |
| 5 bis 60 Vol.-% | H₂O, |
| 8 bis 65 Vol.-% | O₂ und |
| 0,3 bis 20 Vol.-% | H₂. |

Das erfindungsgemäße Verfahren ist aber auch dann günstig, wenn das Reaktionsgasausgangsgemisch 1 0,1 bis 30 Vol.-% CO₂ enthält.

Erfindungsgemäß mögliche Reaktionsgasausgangsgemische 2 können auch enthalten:

| | |
|---|---|
| 3 bis 25 Vol.-% | Acrolein, |
| 5 bis 65 Vol.-% | molekularer Sauerstoff, |
| 6 bis 70 Vol.-% | Propan, |
| 0,3 bis 20 Vol.-% | molekularer Wasserstoff und |
| 8 bis 65 Vol.-% | Wasserdampf. |

Abschließend sei festgehalten, dass die vorliegende Erfindung auch ein Verfahren umfasst, bei dem die in der zweiten Trennzone kristallisativ abgetrennte Acrylsäure aufgeschmolzen wird und sich wenigstens ein Verfahren der radikalischen Polymerisation anschließt, in welchem aufgeschmolzenes Acrylsäurekristallisat zur Herstellung von Polymerisaten radikalisch einpolymerisiert wird. Propan meint in dieser Schrift ohne weiteren Zusatz n-Propan.

Ferner umfasst die vorliegende Erfindung Verfahren, bei denen sich an das erfindungsgemäße Verfahren zur Herstellung von Acrylsäure ein Verfahren zur Herstellung von Acrylsäureestern anschließt, in welchem erfindungsgemäß hergestellte Acrylsäure mit Alkoholen (vorzugsweise Alkanolen, besonders bevorzugt C₁- bis C₁₂-Alkanolen), in der Regel säurekatalysiert, verestert wird. An das Verfahren der Veresterung kann sich wiederum ein Verfahren der radikalischen Polymerisation anschließen, in welchen so hergestellter Acrylsäureester einpolymerisiert wird.

Auch sei nochmals festgehalten, dass wenigstens eine Teilmenge des bei der Überführung (in der Trennzone 1) der Acrylsäure aus dem Produktgasgemisch 2 in die kondensierte Phase verbleibenden Restgases in die erste Reaktionsstufe und/oder in die zweite Reaktionsstufe rückgeführt werden kann.

Auch kann das im Reaktionsgasausgangsgemisch 1 enthaltene Propylen dem Reaktionsgasausgangsgemisch 1 wenigstens teilweise aus einer partiellen Dehydrierung (z. B. homogen und/oder heterogen katalysiert, im Beisein und/oder unter Ausschluss von molekularem Sauerstoff) zugeführt (in der Regel in Begleitung von nicht umgesetztem Propan) werden. In vorstehendem Fall kann wenigstens eine Teilmenge des bei der Überführung der Acrylsäure aus dem Produktgasgemisch 2 in die kondensierte Phase verbleibenden Restgases in die partielle Dehydrierung des Propan rückgeführt werden.

Der Cyclopropangehalt des Reaktionsgasausgangsgemisches 1 kann (wie alle anderen Gehaltes des Reaktionsgasausgangsgemischs 1) problemlos gaschromatographisch ermittelt werden. Durch Analyse von kondensierter Phase des Reaktionsgasausgangsgemisches 1 ist die Nachweisgrenze für Cyclopropan und andere C₃-Kohlenwasserstoffe erweiterbar.

### Beispiele und Vergleichsbeispiele

I. Zweistufige heterogen katalysierte Partialoxidation von Propylen zu Acrylsäure in Abwesenheit und im Beisein von Cyclopropan
A) Allgemeiner Versuchsaufbau der Reaktionsvorrichtung
Reaktor für die erste Oxidationsstufe (1. Reaktionsstufe)
Der Reaktor bestand aus einem doppelwandigen Zylinder aus Edelstahl (zylindrisches Führungsrohr, umgeben von einem zylindrischen Außenbehälter). Die Wanddicken betrugen überall 2 bis 5 mm.
Der Innendurchmesser des äußeren Zylinders betrug 91 mm. Der Innendurchmesser des Führungsrohres betrug ca. 60 mm.
Oben und unten war der doppelwandige Zylinder durch einen Deckel beziehungsweise Boden abgeschlossen.
Das Kontaktrohr (400 cm Gesamtlänge, 26 mm Innendurchmesser, 30 mm Außendurchmesser, 2 mm Wandstärke, Edelstahl) war im Führungsrohr des zylindrischen Behälters so untergebracht, dass es am oberen bzw. unteren Ende desselben (abgedichtet) durch den Deckel bzw. Boden jeweils gerade herausragte. Das Wärmeaustauschmittel (Salzschmelze, bestehend aus 53 Gew.-% Kaliumnitrat, 40 Gew.-% Natriumnitrit und 7 Gew.-% Natriumnitrat) war im zylindrischen Behälter eingeschlossen. Um über die gesamte im zylindrischen Behälter befindliche Kontaktrohrlänge (400 cm) möglichst gleichmäßige thermische Randbedingungen an der Außenwand des Kontaktrohres zu gewährleisten, wurde das Wärmeaustauschmittel mittels einer Propellerpumpe umgepumpt.
Durch eine auf den Außenmantel aufgebrachte elektrische Heizung konnte die Temperatur des Wärmeaustauschmittels auf das gewünschte Niveau geregelt werden. Im übrigen bestand Luftkühlung.

| | |
|---|---|
| Reaktorbeschickung: | Über den Erststufenreaktor betrachtet wurden die Salzschmelze und das Beschickungsgasgemisch des Erststufenreaktors im Gleichstrom geführt. Das Beschickungsgasgemisch trat unten in den Erststufenreaktor ein. Es wurde jeweils mit einer Temperatur von 165°C ins Reaktionsrohr geführt. |
| | Die Salzschmelze trat unten mit einer Temperatur T^{ein} in das zylindrische Führungsrohr ein und oben mit einer Temperatur T^{aus} aus dem zylindrischen Führungsrohr aus, die bis zu 2°C oberhalb von T^{ein} lag. |
| | T^{ein} wurde so eingestellt, dass sich am Ausgang der ersten Oxidationsstufe stets ein Propylenumsatz von 97,8 ± 0,1 mol% bei einfachem Durchgang ergab. |
| Kontaktrohrbeschickung: (von unten nach oben) | Abschnitt A: 90 cm Länge Vorschüttung aus Steatit-Kugeln eines Durchmessers von 4-5 mm. |
| | Abschnitt B: 100 cm Länge Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt C. |
| | Abschnitt C: 200 cm Länge Katalysatorbeschickung mit ringförmigem (5mm x 3 mm x 2 mm = Außendurchmesser x Länge x Innendurchmesser) Vollkatalysator gemäß Beispiel 1 der DE-A 100 46 957 (Stöchiometrie: [Bi₂W₂O₉x2WO₃]_{0,5} [Mo₁₂Co_{5,5}Fe_{2,94}Si_{1,59}K_{0,08}Oₓ]₁). |
| | Abschnitt D: 10 cm Länge Nachschüttung aus Steatit-Ringen der Geometrie 7 mm x 3 mm x 4 mm (Außendurchmesser x Länge x Innendurch-messer) |

Zwischenkühlung und Sauerstoffzwischeneinspeisung (reiner O₂ als Sekundärgas)
Das den ersten Festbettreaktor verlassende Produktgasgemisch 1 wurde zum Zweck der Zwischenkühlung (indirekt mittels Luft) durch ein Verbindungsrohr (40 cm Länge, 26 mm Innendurchmesser, 30 mm Außendurchmesser, 2 mm Wandstärke, Edelstahl, umwickelt mit 1 cm Isoliermaterial) geführt, das auf einer Länge von 20 cm zentriert untergebracht, mit einer Inertschüttung aus Steatit-Ringen der Geometrie 7 mm x 3 mm x 4 mm (Außendurchmesser x Länge x Innendurchmesser) beschickt und unmittelbar an das Erststufenkontaktrohr angeflanscht war.
Das Produktgasgemisch 1 trat stets mit einer Temperatur von > T^{ein} (erste Stufe) in das Verbindungsrohr ein und verließ es mit einer oberhalb von 200 °C und unterhalb von 270 °C gelegenen Temperatur.
Am Ende des Verbindungsrohres wurde dem abgekühlten Produktgasgemisch 1 auf dem Druckniveau des Produktgasgemisches 1 befindlicher molekularer Sauerstoff zudosiert. Das resultierende Gasgemisch (Beschickungsgasgemisch für die zweite Oxidationsstufe) wurde unmittelbar in das Zweitstufenkontaktrohr geführt, an welchem das oben genannte Verbindungsrohr mit seinem anderen Ende ebenfalls angeflanscht war. Die Menge an zudosiertem molekularem Sauerstoff wurde so bemessen, dass das molare Verhältnis von im resultierenden Gasgemisch befindlichen O₂ zu im resultierenden Gasgemisch befindlichen Acrolein 1,3 betrug.
Reaktor für die zweite Oxidationsstufe (2. Reaktionsstufe)
Es wurde ein Kontaktrohr-Festbettreaktor verwendet, der mit jenem für die erste Oxidationsstufe baugleich war. Salzschmelze und Beschickungsgasgemisch wurden über den Reaktor betrachtet im Gleichstrom geführt. Die Salzschmelze trat unten ein, das Beschickungsgasgemisch ebenfalls. Die Eintrittstemperatur T^{ein} der Salzschmelze wurde so eingestellt, dass sich am Ausgang der zweiten Oxidationsstufe stets ein Acroleinumsatz von 99,3 ± 0,1 mol-% bei einfachem Durchgang ergab. T^{aus} der Salzschmelze lag bis zu 2 °C oberhalb von T^{ein}.
Die Kontaktrohrbeschickung (von unten nach oben) war:

| | |
|---|---|
| Abschnitt A: | 70 cm Länge Vorschüttung aus Steatit-Ringen der Geometrie 7 mm x 3 mm x 4 mm |
| | (Außendurchmesser x Länge x Innendurchmesser). |
| Abschnitt B: | 100 cm Länge Katalysatorbeschickung mit einem homogenen Gemisch aus 30 Gew.-% an Steatit-Ringen der Geometrie 7 mm x 3 mm x 4 mm (Außen-durchmesser x Länge x Innendurchmesser) und 70 Gew.-% Schalenkatalysator aus Abschnitt C. |
| Abschnitt C: | 200 cm Länge Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ). |
| Abschnitt D: | 30 cm Länge Nachschüttung aus Steatit-Kugeln eines Durchmessers von 4-5 mm. |

B) In Abhängigkeit von der Zusammensetzung des Reaktionsgasausgangsgemischs 1 der ersten Oxidationsstufe erzielte Ergebnisse (die Propenbelastung wurde auf 150 Nl/l•h eingestellt, die Selektivität der Acrylsäurebildung (bilanziert über beide Reaktionsstufen bezogen auf umgesetztes Propylen) betrug stets ≥ 94 mol-%).
Die Zusammensetzung des Reaktionsgasausgangsgemischs 1 für die erste Oxidationsstufe enthielt im wesentlichen (bezogen auf das Gesamtvolumen des Reaktionsgasausgangsgemischs 1):

| | |
|---|---|
| 6,3 Vol.-% | Propylen, |
| 28 Vol.-% | Propan, |
| X Vol.-% | Cyclopropan, |
| 10,8 Vol.-% | O₂, |
| 5 Vol.-% | H₂O und als Restmenge N₂. |

Aus dem Produktgasgemisch 2 wurde die gebildete Acrylsäure durch Direktkühlung mit vorab gebildetem, auf 4°C abgekühltem und mit Hydrochinon polymerisationsinhibierten, Kondensat auskondensiert. Die nachfolgende Tabelle zeigt den Gewichtsanteil Y der im Kondensat enthaltenen Menge an Propionsäure, bezogen auf die darin enthaltene Menge Acrylsäure in Abhängigkeit von der im Reaktionsgasausgangsgemisch 1 enthaltenen Menge X* an Cyclopropan, hier jedoch angegeben in mol.% relativ zur im Reaktionsgasausgangsgemisch enthaltenen molaren Menge an Propylen.

**Tabelle**

| X* (mol-%) | Y (Gew.ppm) |
|---|---|
| 0,063 | 483 |
| 0,97 | 1114 |

II. Kristallisative Abtrennung aus einer acrylsäurehaltigen flüssigen Phase P
Die flüssige Phase P wies folgende Gehalte auf:

| | |
|---|---|
| 95,201 Gew.-% | Acrylsäure, |
| 0,042 Gew.-% | Methacrylsäure, |
| 0,604 Gew.-% | Benzaldehyd, |
| 0,062 Gew.-% | Propionsäure, |
| 0,687 Gew.-% | Furan-2-aldehyd, |
| 0,663 Gew.-% | Essigsäure, |
| 0,004 Gew.-% | Furan-3-aldehyd, |
| 0,002 Gew.-% | Allylacrylat, |
| 0,009 Gew.-% | Acrolein und |
| 2,20 Gew.-% | Wasser. |

Sie war durch Zusatz von 150 Gew.ppm Monomethylether des Hydrochinons (MEHQ) und < 1000 Gew.ppm Phenotziazin (bezogen auf enthaltene Acrylsäure) polymerisationsinhibiert.
1800 g der flüssigen Phase P wurden in einen gerührten Metallkessel (2 l Innenvolumen, nahezu wandgängiger Wendelrührer) gefüllt.
Mit einer Abkühlgeschwindigkeit von 1 K/h wurde die Temperatur der im Mantel geführten Kühlflüssigkeit (Wasser/Glykol-Mischung) so lange abgesenkt, bis die resultierende Kristallisatsuspension (Acrylsäurekristalle suspendiert in Restschmelze) einen Feststoffgehalt von 18 Gew.-% aufwies. Dann wurde ein Teil der Kristallisatsuspension entnommen und auf einer Laborzentrifuge in einem mit einem Polypropylenfiltergewebe ausgerüsteten Siebbecher bei 2000 Umdrehungen je Minute 180 Sekunden zentrifugiert und die so verbliebene Mutterlauge nahezu vollständig abgeschleudert. Analyse des verbliebenen Kristallisats sowie der abgeschleuderten Mutterlauge ergab für die Propionsäure den Abreicherungskoeffizienten 3,9 (der Abreicherunskoeffizient ist das Mengenverhältnis von in der Mutterlauge verbliebener Propionsäure zu im Kristallisat verbliebener Propionsäure, jeweils ausgedrückt als Gew.-% bezogen auf die Gesamtmenge an Mutterlauge bzw. die Gesamtmenge an Kristallisat).
US Provisional Patent Application No. 60/752,362, wurde am 22.12.2005 eingereicht.

## Patentansprüche

1. Verfahren der heterogen katalysierten partiellen Gasphasenoxidation von Propylen zu Acrylsäure, bei dem man in einer ersten Reaktionszone ein Propylen, molekularen Sauerstoff und wenigstens ein inertes Verdünnungsgas enthaltendes Reaktionsgasausgangsgemisch 1, das den molekularen Sauerstoff und das Propylen in einem molaren Verhältnis O₂ : C₃H₆ ≥ 1 enthält, zunächst in einer ersten Reaktionsstufe bei erhöhter Temperatur so durch wenigstens ein erstes Katalysatorbett führt, dessen Katalysatoren als Aktivmasse wenigstens ein Mo, Fe und Bi enthaltendes Multimetalloxid aufweisen, dass der Propylenumsatz bei einmaligem Durchgang durch das Katalysatorbett ≥ 90 mol-% und die damit einhergehenden Selektivität S^{AC} der Acroleinbildung sowie der Acrylsäurenebenproduktbildung zusammen ≥ 80 mol-% betragen, die Temperatur des die erste Reaktionsstufe verlassenden Produktgasgemischs 1 durch direkte Kühlung, oder durch indirekte Kühlung, oder durch direkte und indirekte Kühlung gegebenenfalls verringert und dem Produktgasgemisch 1 gegebenenfalls Sekundärgas in Form von molekularem Sauerstoff, oder Inertgas, oder molekularem Sauerstoff und Inertgas zugibt, und danach das Produktgasgemisch 1 als Acrolein, molekularen Sauerstoff und wenigstens ein inertes Verdünnungsgas enthaltendes Reaktionsgasausgangsgemisch 2, das den molekularen Sauerstoff und das Acrolein in einem molaren Verhältnis O₂ : C₃H₄O ≥ 0,5 enthält, in einer zweiten Reaktionsstufe bei erhöhter Temperatur so durch wenigsten ein zweites Katalysatorbett führt, dessen Katalysatoren als Aktivmasse wenigstens ein Mo und V enthaltendes Multimetalloxid aufweisen, dass der Acroleinumsatz bei einmaligem Durchgang durch das Katalysatorbett ≥ 90 mol-% und die Selektivität S^{AA} der über beide Reaktionsstufen bilanzierten Acrylsäurebildung, bezogen auf umgesetztes Propylen, ≥ 70 mol-% beträgt, anschließend aus dem in der zweiten Reaktionsstufe gebildeten Produktgasgemisch 2 die darin enthaltene Acrylsäure in einer ersten Trennzone in die kondensierte Phase überführt und danach in einer zweiten Trennzone die Acrylsäure aus der kondensierten Phase durch Anwendung wenigstens eines thermischen Trennverfahrens abtrennt,
**dadurch gekennzeichnet,**
**dass** das Reaktionsgasausgangsgemisch 1, bezogen auf die darin enthaltene molare Menge an Propylen, bis zu 3 mol-% Cyclopropan enthält und das wenigstens eine thermische Trennverfahren in der zweiten Trennzone wenigstens eine kristallisative Abtrennung von Acrylsäure umfasst.

2. Verfahren nach Anspruch 1, **dadurch gekennzeichnet, dass** das Reaktionsgasausgangsgemisch 1, bezogen auf die darin enthaltene molare Menge an Propylen, 50 molppb bis 2 mol-% Cyclopropan enthält.

3. Verfahren nach Anspruch 1, **dadurch gekennzeichnet, dass** das Reaktionsgasausgangsgemisch 1, bezogen auf die darin enthaltene molare Menge an Propylen, 100 molppb bis 1 mol-% Cyclopropan enthält.

4. Verfahren nach einem der Ansprüche 1 bis 3, **dadurch gekennzeichnet, dass** die Acrylsäure durch absorptive Maßnahmen aus dem Produktgasgemisch 2 in die kondensierte Phase überführt wird.

5. Verfahren nach einem der Ansprüche 1 bis 3, **dadurch gekennzeichnet, dass** die Acrylsäure durch kondensative Maßnahmen aus dem Produktgasgemisch 2 in die kondensierte Phase überführt wird.

6. Verfahren nach einem der Ansprüche 1 bis 3, **dadurch gekennzeichnet, dass** die Acrylsäure durch absorptive und kondensative Maßnahmen aus dem Produktgasgemisch 2 in die kondensierte Phase überführt wird.

7. Verfahren nach einem der Ansprüche 4 oder 6, **dadurch gekennzeichnet, dass** als Absorptionsmittel Wasser oder eine wässrige Lösung verwendet wird.

8. Verfahren nach einem der Ansprüche 1 bis 3, **dadurch gekennzeichnet, dass** die Acrylsäure durch fraktionierende Kondensation aus dem Produktgasgemisch 2 in die kondensierte Phase überführt wird.

9. Verfahren nach einem der Ansprüche 1 bis 8, **dadurch gekennzeichnet, dass** wenigstens eine kristallisative Abtrennung der Acrylsäure in der zweiten Trennzone aus einer bei der in der ersten Trennzone durchgeführten Überführung der Acrylsäure aus dem Produktgasgemisch 2 in die kondensierte Phase anfallenden acrylsäurehaltigen kondensierten Phase heraus erfolgt.

10. Verfahren nach einem der Ansprüche 1 bis 8, **dadurch gekennzeichnet, dass** wenigstens eine kristallisative Abtrennung der Acrylsäure in der zweiten Trennzone aus einer flüssigen Phase heraus erfolgt, die das Ergebnis einer Anwendung von wenigstens einem von einer Kristallisation verschiedenen thermischen Trennverfahren auf die in der ersten Trennzone anfallenden acrylsäurehaltigen kondensierten Phase ist.

11. Verfahren nach einem der Ansprüche 1 bis 10, **dadurch gekennzeichnet, dass** die wenigstens eine kristallisative Abtrennung der Acrylsäure als fraktionierte Kristallisation durchgeführt wird.

12. Verfahren nach einem der Ansprüche 1 bis 11, **dadurch gekennzeichnet, dass** die wenigstens eine kristallisative Abtrennung der Acrylsäure als Schichtkristallisation ausgeführt wird.

13. Verfahren nach einem der Ansprüche 1 bis 12, **dadurch gekennzeichnet, dass** die wenigstens eine kristallisative Abtrennung der Acrylsäure eine Kombination von dynamischer und statischer Schichtkristallisation ist.

14. Verfahren nach einem der Ansprüche 1 bis 11, **dadurch gekennzeichnet, dass** die wenigstens eine kristallisative Abtrennung der Acrylsäure als Suspensionskristallisation ausgeführt wird.

15. Verfahren nach Anspruch 14, **dadurch gekennzeichnet, dass** die Trennung von gebildetem Suspensionskristallisat und verbliebener Mutterlauge in einer Waschkolonne durchgeführt wird.

16. Verfahren nach einem der Ansprüche 1 bis 15, **dadurch gekennzeichnet, dass** die wenigstens eine kristallisative Abtrennung von Acrylsäure aus einer flüssigen Phase heraus erfolgt, die Wasser enthält.

17. Verfahren nach einem der Ansprüche 1 bis 16, **dadurch gekennzeichnet, dass** die in der zweiten Trennzone kristallisativ abgetrennte Acrylsäure aufgeschmolzen wird und sich wenigstens ein Verfahren der radikalischen Polymerisation anschließt, in welchem aufgeschmolzenes Acrylsäurekristallisat zur Herstellung von Polymerisaten radikalisch einpolymerisiert wird.

18. Verfahren nach einem der Ansprüche 1 bis 17, **dadurch gekennzeichnet, dass** die wenigstens eine kristallisative Abtrennung der Acrylsäure in der zweiten Trennzone mit wenigstens einem unscharfen Trennverfahren in der ersten und/oder zweiten Trennzone dadurch rückgekoppelt wird, dass man die bei der kristallisativen Acrylsäureabtrennung verbleibende Mutterlauge wenigstens teilweise in wenigstens eines der unscharfen Trennverfahren rückführt.

19. Verfahren nach einem der Ansprüche 1 bis 18, **dadurch gekennzeichnet, dass** das wenigstens eine Mo, Fe und Bi enthaltende Multimetalloxid ein solches der allgemeinen Formel IV,
Mo₁₂BiₐFe_{b}X_{c}¹X_{d}²Xₑ³X_{f}⁴Oₙ , (IV)
mit
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Alumium, Titan und/oder Zirkonium,
a = 0,5 bis 5,
b = 0,01 bis 5,
c = 0 bis 10,
d = 0 bis 2,
e = 0 bis 8,
f = 0 bis 10 und
n = eine Zahl, die durch die Wertigkeit und Häufigkeit der von Sauerstoff verschiedenen Elemente in IV bestimmt wird,
ist.

20. Verfahren nach einem der Ansprüche 1 bis 19, **dadurch gekennzeichnet, dass** das wenigstens eine Mo und V enthaltende Multimetalloxid ein solches der allgemeinen Formel VII,
Mo₁₂VₐX_{b}¹X_{c}²X_{d}³Xₑ⁴X_{f}⁵X_{g}⁶Oₙ , (VII)
mit
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in VII bestimmt wird,
ist.

21. Verfahren nach einem der Ansprüche 1 bis 20, **dadurch gekennzeichnet, dass** die volumenspezifische Aktivität des wenigstens einen ersten Katalysatorbetts in Strömungsrichtung des Reaktionsgasausgangsgemischs 1 über die Länge des Strömungsweges wenigstens einmal zunimmt.

22. Verfahren nach einem der Ansprüche 1 bis 21, **dadurch gekennzeichnet, dass** die volumenspezifische Aktivität des wenigstens einen zweiten Katalysatorbetts in Strömungsrichtung des Reaktionsgasausgangsgemischs 2 über die Länge des Strömungsweges wenigstens einmal zunimmt.

23. Verfahren nach einem der Ansprüche 1 bis 22, **dadurch gekennzeichnet, dass** das wenigstens eine erste Katalysatorbett ein Festbett ist und seine Propenbelastung ≥ 120 Nl/l•h und ≤ 250 Nl/l•h beträgt.

24. Verfahren nach einem der Ansprüche 1 bis 23, **dadurch gekennzeichnet, dass** das Reäktionsgasausgangsgemisch 1 6 bis 13 Vol.-% Propylen enthält.

25. Verfahren nach einem der Ansprüche 1 bis 24, **dadurch gekennzeichnet, dass** das Reaktionsgasausgangsgemisch 1 > 0 bis 35 Vol.-% H₂O enthält.

26. Verfahren nach einem der Ansprüche 1 bis 25, **dadurch gekennzeichnet, dass** das Reaktionsgasausgangsgemisch 1 ≥ 0,01 Vol.-% Propan enthält.

27. Verfahren nach einem der Ansprüche 1 bis 26, **dadurch gekennzeichnet, dass** wenigstens eine Teilmenge des bei der Überführung der Acrylsäure aus dem Produktgasgemisch 2 in die kondensierte Phase verbleibenden Restgas in die erste Reaktionsstufe und/oder in die zweite Reaktionsstufe rückgeführt wird.

28. Verfahren nach einem der Ansprüche 1 bis 27, **dadurch gekennzeichnet, dass** das im Reaktionsgasausgangsgemisch 1 enthaltene Propylen dem Reaktionsgasausgangsgemisch 1 wenigstens teilweise aus einer partiellen Dehydrierung von Propan zugeführt wird.

29. Verfahren nach Anspruch 28, **dadurch gekennzeichnet, dass** wenigstens eine Teilmenge des bei der Überführung der Acrylsäure aus dem Produktgasgemisch 2 in die kondensierte Phase verbleibenden Restgas in die partielle Dehydrierung des Propan rückgeführt wird.

## Claims

1. A process for heterogeneously catalyzed partial gas phase oxidation of propylene to acrylic acid, in which, in a first reaction zone, a starting reaction gas mixture 1 which comprises propylene, molecular oxygen and at least one inert diluent gas and comprises the molecular oxygen and the propylene in a molar O₂ : C₃H₆ ratio of ≥ 1, in a first reaction stage at elevated temperature, is first conducted through at least one first catalyst bed whose catalysts have at least one multimetal oxide comprising Mo, Fe and Bi as the active composition, such that the propylene conversion in single pass through the catalyst bed is ≥ 90 mol% and the accompanying selectivity S^{AC} of acrolein formation and of acrylic acid by-product formation together is ≥ 80 mol%, the temperature of the product gas mixture 1 leaving the first reaction stage is optionally reduced by direct cooling or by indirect cooling or by direct and indirect cooling, and secondary gas in the form of molecular oxygen or inert gas or molecular oxygen and inert gas is optionally added to product gas mixture 1, and then product gas mixture 1, as a starting reaction gas mixture 2 which comprises acrolein, molecular oxygen and at least one inert diluent gas and comprises the molecular oxygen and the acrolein in a molar O₂ : C₃H₄O ratio of ≥ 0.5, in a second reaction stage at elevated temperature, is conducted through at least one second catalyst bed whose catalysts have at least one multimetal oxide comprising Mo and V as the active composition, such that the acrolein conversion in single pass through the catalyst bed is ≥ 90 mol% and the selectivity S^{AA} of acrylic acid formation assessed over both reaction stages, based on propylene converted, is ≥ 70 mol%, then the acrylic acid present in the product gas mixture 2 formed in the second reaction stage, in a first separation zone, is converted therefrom to the condensed phase and then, in a second separation zone, the acrylic acid is removed from the condensed phase by use of at least one thermal separation process,
wherein
starting reaction gas mixture 1, based on the molar amount of propylene present therein, comprises up to 3 mol% of cyclopropane and the at least one thermal separation process in the second separation zone comprises at least one crystallizative removal of acrylic acid.

2. The process according to claim 1, wherein starting reaction gas mixture 1, based on the molar amount of propylene present therein, comprises from 50 molppb to 2 mol% of cyclopropane.

3. The process according to claim 1, wherein starting reaction gas mixture 1, based on the molar amount of propylene present therein, comprises from 100 molppb to 1 mol% of cyclopropane.

4. The process according to any of claims 1 to 3, wherein the acrylic acid is converted from product gas mixture 2 into the condensed phase by absorptive measures.

5. The process according to any of claims 1 to 3, wherein the acrylic acid is converted from product gas mixture 2 into the condensed phase by condensative measures.

6. The process according to any of claims 1 to 3, wherein the acrylic acid is converted from product gas mixture 2 into the condensed phase by absorptive and condensative measures.

7. The process according to any of claims 4 to 6, wherein the absorbent used is water or an aqueous solution.

8. The process according to any of claims 1 to 3, wherein the acrylic acid is converted from product gas mixture 2 into the condensed phase by fractional condensation.

9. The process according to any of claims 1 to 8, wherein at least one crystallizative removal of the acrylic acid is effected in the second separation zone out of an acrylic acid-containing condensed phase obtained in the conversion of acrylic acid from product gas mixture 2 into the condensed phase carried out in the first separation zone.

10. The process according to any of claims 1 to 8, wherein at least one crystallizative removal of the acrylic acid is effected in the second separation zone out of a
liquid phase which is the result of use of at least one thermal separation process other than a crystallization on the acrylic acid-containing condensed phase obtained in the first separation zone.

11. The process according to any of claims 1 to 10, wherein the at least one crystallizative removal of the acrylic acid is carried out as a fractional crystallization.

12. The process according to any of claims 1 to 11, wherein the at least one crystallizative removal of the acrylic acid is performed as a layer crystallization.

13. The process according to any of claims 1 to 12, wherein the at least one crystallizative removal of acrylic acid is a combination of dynamic and static layer crystallization.

14. The process according to any of claims 1 to 11, wherein the at least one crystallizative removal of the acrylic acid is performed as a suspension crystallization.

15. The process according to claim 14, wherein the separation of suspension crystals formed and mother liquor remaining is carried out in a wash column.

16. The process according to any of claims 1 to 15, wherein the at least one crystallizative removal of acrylic acid is effected out of a liquid phase which comprises water.

17. The process according to any of claims 1 to 16, wherein the acrylic acid removed by crystallization in the second separation zone is melted and at least one process for free-radical polymerization follows, in which molten acrylic acid crystals are free-radically polymerized to prepare polymers.

18. The process according to any of claims 1 to 17, wherein the at least one crystallizative removal of acrylic acid in the second separation zone is coupled with at least one indistinct separation process in the first and/or second separation zone by at least partly recycling the mother liquor remaining in the crystallizative acrylic acid removal into at least one of the indistinct separation processes.

19. The process according to any of claims 1 to 18, wherein the at least one multimetal oxide comprising Mo, Fe and Bi is one of the general formula IV
Mo₁₂BiₐFe_{b}¹X_{d}²Xₑ³X_{f}⁴Oₙ (IV)
where
X¹ = nickel and/or cobalt,
X² = thallium, an alkali metal and/or an alkaline earth metal,
X³ = zinc, phosphorus, arsenic, boron, antimony, tin, cerium, a lead and/or tungsten,
X⁴ = silicon, aluminum, titanium and/or zirconium,
a = from 0.5 to 5,
b = from 0.01 to 5,
c = from 0 to 10,
d = from 0 to 2,
e = from 0 to 8,
f = from 0 to 10 and
n = a number which is determined by the valency and frequency of the elements in IV other than oxygen

20. The process according to any of claims 1 to 19, wherein the at least one multimetal oxide comprising Mo and V is one of the general formula VII
Mo₁₂VₐXb¹X_{c}²X_{d}³Xₑ⁴X_{f}⁵X_{g}⁶Oₙ (VII)
where
X¹ = W, Nb, Ta, Cr and/or Ce,
X² = Cu, Ni, Co, Fe, Mn and/or Zn,
X³ = Sb and/or Bi,
X⁴ = one or more alkali metals,
X⁵ = one or more alkaline earth metals,
X⁶ = Si, Al, Ti and/or Zr,
a = 1 to 6,
b = 0.2 to 4,
c = 0.5 to 18,
d = 0 to 40,
e = 0 to 2,
f = 0 to 4,
g = 0 to 40 and
n = a number which is determined by the valency and frequency of the elements in VII other than oxygen.

21. The process according to any of claims 1 to 20, wherein the volume-specific activity of the at least one first catalyst bed increases at least once over the length of the flow path in flow direction of starting reaction gas mixture 1.

22. The process according to any of claims 1 to 21 wherein the volume-specific activity of the at least one second catalyst bed increases at least once over the length of the flow path in flow direction of starting reaction gas mixture 2.

23. The process according to any of claims 1 to 22, wherein the at least one first catalyst bed is a fixed bed and its propene loading is ≥ 120 1 (STP)/l•h and ≤ 250 1 (STP)/l•h.

24. The process according to any of claims 1 to 23, wherein starting reaction gas mixture 1 comprises from 6 to 13% by volume of propylene.

25. The process according to any of claims 1 to 24, wherein starting reaction gas mixture 1 comprises from > 0 to 35% by volume of H₂O.

26. The process according to any of claims 1 to 25, wherein starting reaction gas mixture 1 comprises ≥ 0.01% by volume of propane.

27. The process according to any of claims 1 to 26, wherein at least a portion of the residual gas remaining in the conversion of acrylic acid from product gas mixture 2 into the condensed phase is recycled into the first reaction stage and/or into the second reaction stage.

28. The process according to any of claims 1 to 27, wherein the propylene present in starting reaction gas mixture 1 is supplied to starting reaction gas mixture 1 at least partly from a partial dehydrogenation of propane.

29. The process according to claim 28, wherein at least a portion of the residual gas remaining in the conversion of acrylic acid from product gas mixture 2 into the condensed phase is recycled into the partial dehydrogenation of propane.

## Revendications

1. Procédé pour l'oxydation en phase gazeuse partielle de propylène en acide acrylique, à catalyse hétérogène, dans lequel dans une première zone de réaction on fait passer à travers au moins un premier lit de catalyseurs, dont les catalyseurs comportent en tant que masse active au moins un oxyde multimétallique contenant Mo, Fe et Bi, d'abord dans un premier stade de réaction, un mélange initial de gaz de réaction 1 contenant du propylène, de l'oxygène moléculaire et au moins un gaz inerte de dilution, qui contient l'oxygène moléculaire et le propylène en un rapport molaire O₂ : C₃H₆ ≥ 1, à température élevée, de manière que le taux de conversion du propylène lors d'un seul passage à travers le lit de catalyseurs soit ≥ 90 % en moles et la sélectivité S^{AC} qui l'accompagne de la formation d'acroléine ainsi que de la formation de sous-produits d'acide acrylique soient ensemble ≥ 80 % en moles, la température du mélange de gaz produit 1 quittant le premier stade de réaction est éventuellement abaissée par refroidissement direct, ou par refroidissement indirect, ou par refroidissement direct et indirect et on ajoute au mélange de gaz produit 1 éventuellement du gaz secondaire sous forme d'oxygène moléculaire, ou un gaz inerte, ou de l'oxygène moléculaire et un gaz inerte, et ensuite, dans un deuxième stade de réaction on fait passer à travers un deuxième lit de catalyseurs, dont les catalyseurs comportent en tant que masse active au moins un oxyde multimétallique contenant Mo et V, le mélange de gaz produit 1, en tant que mélange initial de gaz de réaction 2 contenant de l'acroléine, de l'oxygène moléculaire et au moins un gaz inerte de dilution, qui contient l'oxygène moléculaire et l'acroléine en un rapport molaire O₂ : C₃H₄O ≥ 0,5, à température élevée, de manière que le taux de conversion de l'acroléine lors d'un seul passage à travers le lit de catalyseurs soit ≥ 90 % en moles et la sélectivité S^{AA} de la formation d'acide acrylique, établie sur les deux stades de réaction, par rapport au propylène mis en réaction, soit ≥ 70 % en moles, puis dans une première zone de séparation on transfère dans la phase condensée, à partir du mélange de gaz produit 2 formé dans le deuxième stade de réaction l'acide acrylique contenu dans celui-ci et ensuite dans une deuxième zone de séparation on sépare par application d'au moins un procédé de séparation thermique l'acide acrylique de la phase condensée,
**caractérisé en ce que**
le mélange initial de gaz de réaction 1 contient, par rapport à la quantité molaire de propylène contenue dans celui-ci, jusqu'à 3 % en moles de cyclopropane et ledit au moins un procédé de séparation thermique dans la deuxième zone de séparation comprend au moins une séparation d'acide acrylique par cristallisation.

2. Procédé selon la revendication 1, **caractérisé en ce que** le mélange initial de gaz de réaction 1 contient, par rapport à la quantité molaire de propylène contenue dans celui-ci, de 50 ppb en moles à 2 % en moles de cyclopropane.

3. Procédé selon la revendication 1, **caractérisé en ce que** le mélange initial de gaz de réaction 1 contient, par rapport à la quantité molaire de propylène contenue dans celui-ci, de 100 ppb en moles à 1 % en moles de cyclopropane.

4. Procédé selon l'une quelconque des revendications 1 à 3, **caractérisé en ce qu'**on transfère par des moyens d'absorption l'acide acrylique, du mélange de gaz produit 2, dans la phase condensée.

5. Procédé selon l'une quelconque des revendications 1 à 3, **caractérisé en ce qu'**on transfère par des moyens de condensation l'acide acrylique, du mélange de gaz produit 2, dans la phase condensée.

6. Procédé selon l'une quelconque des revendications 1 à 3, **caractérisé en ce que** qu'on transfère par des moyens d'absorption et de condensation l'acide acrylique, du mélange de gaz produit 2, dans la phase condensée.

7. Procédé selon l'une quelconque des revendications 4 et 6, **caractérisé en ce qu'**on utilise comme absorbant l'eau ou une solution aqueuse.

8. Procédé selon l'une quelconque des revendications 1 à 3, **caractérisé en ce qu'**on transfère l'acide acrylique par condensation fractionnée l'acide acrylique, du mélange de gaz produit 2, dans la phase condensée.

9. Procédé selon l'une quelconque des revendications 1 à 8, **caractérisé en ce qu'**au moins une séparation de l'acide acrylique par cristallisation s'effectue dans la deuxième zone de séparation à partir d'une phase condensée contenant de l'acide acrylique, produite lors du transfert de l'acide acrylique, du mélange de gaz produit 2, dans la phase condensée, effectué dans la première zone de séparation.

10. Procédé selon l'une quelconque des revendications 1 à 8, **caractérisé en ce qu'**au moins une séparation de l'acide acrylique par cristallisation s'effectue dans la deuxième zone de séparation à partir d'une phase liquide qui est le résultat d'une application d'au moins un procédé de séparation thermique différent d'une cristallisation à la phase condensée contenant de l'acide acrylique, produite dans la première zone de séparation.

11. Procédé selon l'une quelconque des revendications 1 à 10, **caractérisé en ce que** ladite au moins une séparation de l'acide acrylique par cristallisation est effectuée en tant que cristallisation fractionnée.

12. Procédé selon l'une quelconque des revendications 1 à 11, **caractérisé en ce que** ladite au moins une séparation de l'acide acrylique par cristallisation est effectuée en tant que cristallisation en couches.

13. Procédé selon l'une quelconque des revendications 1 à 12, **caractérisé en ce que** ladite au moins une séparation de l'acide acrylique par cristallisation est une combinaison de cristallisation dynamique et de cristallisation statique.

14. Procédé selon l'une quelconque des revendications 1 à 11, **caractérisé en ce que** ladite au moins une séparation de l'acide acrylique par cristallisation est effectuée en tant que cristallisation en suspension.

15. Procédé selon la revendication 14, **caractérisé en ce que** la séparation du cristallisat en suspension formé et de la liqueur mère restante est effectuée dans une colonne de lavage.

16. Procédé selon l'une quelconque des revendications 1 à 15, **caractérisé en ce que** ladite au moins une séparation de l'acide acrylique par cristallisation s'effectue à partir d'une phase liquide qui contient de l'eau.

17. Procédé selon l'une quelconque des revendications 1 à 16, **caractérisé en ce qu'**on fait fondre l'acide acrylique séparé par cristallisation dans la deuxième zone de séparation et qu'à cela fait suite au moins un procédé de la polymérisation radicalaire, dans lequel du cristallisat d'acide acrylique fondu est incorporé par polymérisation radicalaire pour la production de polymérisats.

18. Procédé selon l'une quelconque des revendications 1 à 17, **caractérisé en ce qu'**on recombine ladite au moins une séparation de l'acide acrylique par cristallisation dans la deuxième zone de séparation avec au moins un procédé de séparation sans netteté dans la première et/ou la deuxième zone de séparation, en renvoyant au moins en partie dans au moins l'un des procédés de séparation sans netteté la liqueur mère restant lors de la séparation d'acide acrylique par cristallisation.

19. Procédé selon l'une quelconque des revendications 1 à 18, **caractérisé en ce que** ledit au moins un oxyde multimétallique contenant Mo, Fe et Bi est un tel oxyde de formule générale IV,
Mo₁₂BiₐFe_{b}X_{c}¹X_{d}²Xₑ³X_{f}⁴Oₙ (IV)
où
X¹ = nickel et/ou cobalt,
X² = thallium, un métal alcalin et/ou un métal alcaline-terreux,
X³ = zinc, phosphore, arsenic, bore, antimoine, étain, cérium, plomb et/ou tungstène,
X⁴ = silicium, aluminium, titane et/ou zirconium,
a = 0,5 à 5,
b = 0,01 à 5,
c = 0 à 10,
d = 0 à 2,
e = 0 à 8,
f = 0 à 10 et
n = un nombre qui est déterminé par la valence et la fréquence des éléments différents de l'oxygène dans IV.

20. Procédé selon l'une quelconque des revendications 1 à 19, **caractérisé en ce que** ledit au moins un oxyde multimétallique contenant Mo et V est un tel oxyde de formule générale VII,
Mo₁₂VₐX_{b}¹X_{c}²X_{d}³Xₑ⁴X_{f}⁵X_{g}⁶Oₙ (VII)
où
X¹ = W, Nb, Ta, Cr et/ou Ce,
X² = Cu, Ni, Co, Fe, Mn et/ou Zn,
X³ = Sb et/ou Bi,
X⁴ = un ou plusieurs métaux alcalins,
X⁵ = un ou plusieurs métaux alcalino-terreux,
X⁶ = Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40 et
n = un nombre qui est déterminé par la valence et la fréquence des éléments différents de l'oxygène dans VII.

21. Procédé selon l'une quelconque des revendications 1 à 20, **caractérisé en ce que** l'activité spécifique volumique dudit au moins un premier lit de catalyseurs dans le sens de l'écoulement du mélange initial de gaz de réaction 1 augmente au moins une fois sur la longueur de la voie d'écoulement.

22. Procédé selon l'une quelconque des revendications 1 à 21, **caractérisé en ce que** l'activité spécifique volumique dudit au moins un deuxième lit de catalyseur dans le sens de l'écoulement du mélange initial de gaz de réaction 2 augmente au moins une fois sur la longueur de la voie d'écoulement.

23. Procédé selon l'une quelconque des revendications 1 à 22, **caractérisé en ce qu'**au moins un premier lit de catalyseurs est un lit fixe et sa charge en propène est ≥ 120 l normaux/l.h et ≤ 250 l normaux/l.h.

24. Procédé selon l'une quelconque des revendications 1 à 23, **caractérisé en ce que** le mélange initial de gaz de réaction 1 contient de 6 à 13 % en volume de propylène.

25. Procédé selon l'une quelconque des revendications 1 à 24, **caractérisé en ce que** le mélange initial de gaz de réaction 1 contient de > 0 à 35 % en volume de H₂O.

26. Procédé selon l'une quelconque des revendications 1 à 25, **caractérisé en ce que** le mélange initial de gaz de réaction 1 contient ≥ 0,01 % en volume de propane.

27. Procédé selon l'une quelconque des revendications 1 à 26, **caractérisé en ce qu'**au moins une quantité partielle du gaz résiduel restant lors du transfert de l'acide acrylique, du mélange de gaz produit 2, dans la phase condensée, est renvoyée dans le premier stade de réaction et/ou dans le deuxième stade de réaction.

28. Procédé selon l'une quelconque des revendications 1 à 27, **caractérisé en ce que** le propylène contenu dans le mélange initial de gaz de réaction 1 est envoyé au moins en partie à partir d'une déshydrogénation partielle du propane, au mélange initial de gaz de réaction 1.

29. Procédé selon la revendication 28, **caractérisé en ce qu'**au moins une quantité partielle du gaz résiduel restant lors du transfert de l'acide acrylique, du mélange de gaz produit 2, dans la phase condensée, est recyclée dans la déshydrogénation partielle du propane.
